# EUROPEAN PATENT APPLICATION

(11) **EP 4 471 047 A1**
(43) Date of publication of application: **04.12.2024**
(21) Application number: 23176557.9
(22) Date of filing: 31.05.2023
(51) Int. Cl.: C07K 5/062, C07K 5/065, A61K 38/00, A23L 33/10, A61K 31/444, A61K 31/06, A61K 47/52, A61K 9/00, A61K 9/14, A61P 1/00, A61P 1/04, A61P 17/02, A61P 29/00, C07D 471/04, A61K 33/06

(54) **MODULATORS OF SORTILIN ACTIVITY**

(71) Applicant: Insusense ApS, 2100 København (DK)
(72) Inventor: LITTLE, Paul Brian, 2100 Copenhagen (DK); CASES-THOMAS, Manuel Javier, 2100 Copenhagen (DK); KJØLBY, Mads Fuglsang, 2100 Copenhagen (DK); NYKJÆR, Anders, 2100 Copenhagen (DK)
(74) Representative: Gill Jennings & Every LLP

(57) **Abstract**

The present invention relates to compounds of formula (I), which are modulators of sortilin activity. The invention also relates to pharmaceutical compositions comprising these compounds and to the use of these compounds in the treatment or prevention of medical conditions where modulation of sortilin activity is beneficial.

## Description

### FIELD OF THE INVENTION

The present invention relates to compounds of formula (I), which are modulators of sortilin activity. The invention also relates to pharmaceutical compositions comprising these compounds and to the use of these compounds in the treatment or prevention of medical conditions where modulation of sortilin activity is beneficial. Such medical conditions include a neurodegenerative disorder, an inflammatory disorder, a lysosomal storage disorder, a cancer, pain, diabetes mellitus, diabetic retinopathy, glaucoma, uveitis, cardiovascular disease, hereditary eye conditions or hearing loss.

### BACKGROUND

Sortilin (encoded by *SORT1*) is a type 1 membrane receptor in the vacuolar protein sorting 10 protein (VPS10P) family of sorting receptors, and is abundantly expressed in the central nervous system, the inner ear, and in some peripheral tissues involved in metabolic control^{1,2,3,4}. Sortilin has an amino acid sequence according to SEQ ID NO: 1 and comprises a signal peptide, a propeptide, the Vps10p domain, a 10cc domain (10CCa + 10CCb), a transmembrane domain and a large cytoplasmic tail. The luminal domain of sortilin has 6 potential N-linked glycosylation sites, whilst the cytoplasmic tail enables for the recruitment of various adapter proteins.

Sortilin binds to a vast number of ligands and membrane receptors and as a result engages in functions known to be important in cellular signalling and sorting. For example, sortilin is involved in signalling by proneurotrophins: the proforms of nerve growth factor (proNGF), brain derived neurotrophic factor (proBDNF), and neurotrophin-3 (proNT3), respectively. In complex with the protein p75NTR (p75 neurotrophin receptor), sortilin has been reported to form the receptor for proneurotrophin-mediated apoptotic effects leading to degeneration and cell death in cellular and animal models^{5,6,7}.

Previous work has suggested a role for sortilin in cellular sorting and signalling associated with diseases such as diabetes and obesity (Huang et a12013 Mol Biol Cell Oct;24(19):3115-22)⁸. Sortilin facilitates translocation of GLUT4 to the plasma membrane and rescues it from degradation in the lysosomes (Pan et al Mol Biol Cell. 2017 Jun 15;28(12):1667-1675)⁹. Sortilin levels have been shown to be modulated by the level of inflammation associated with these diseases. The proinflammatory cytokine, TNFα, reduces both mRNA levels and protein levels of sortilin in cultured mouse and human adipocytes, as well as *in vivo* when injected into mice (Kaddai et al. Diabetologia 52: 932-40, 2009)¹⁰. Sortilin can also influence cytokine secretion: targeting sortilin in immune cells has been proposed to attenuate inflammation and reduce atherosclerosis disease progression (Mortensen et al. J Clin Invest 124(12):5317-22, 2014)¹¹. Additionally, US 2016/0331746 describes various scaffolds of small molecules capable of binding to the active site of sortilin. Sortilin is involved in the regulation of glucose uptake (Shi & Kandror. Developmental Cell 9:99-108, 2005)¹² and the development of lipid disorder diseases (Gao et al. DNA and Cell Biology 36(12):1050-61, 2017)¹³.

Further, plasma sortilin levels have been reported to be a potential biomarker for identifying patients with either coronary heart disease or diabetes mellitus (Oh et al. Cardiovascular Diabetology 16:92, 2017)¹⁴. Patients that showed increased sortilin levels within their plasma, and therefore identifiable as suffering from the above conditions, also displayed enhanced glucose levels suggesting sortilin as a therapeutic target for treating these conditions.

Previous work by the current inventors to devise compounds which can modulate sortilin activity are disclosed in European Application No. EP21170274.1 and its corresponding PCT application No. PCT/EP2022/060742; and European Application No. EP21173972.7 and its corresponding PCT Application No. PCT/EP2022/063049, the entire contents of which are herein incorporated by reference.

In addition to effectively targeting and binding to sortilin, compounds useful for modulating sortilin activity in a subject preferably have optimised pharmacokinetics. For example, this may equate to prolonged half-life *in-vivo* or improved delivery to target tissues. Improved persistence of such compounds in a subject is an important clinical parameter. This determines the required dosages and frequency of dosing of the compounds, as well as formulation requirements needed to achieve the desired therapeutic effect upon administration.

An increase in the *in-vivo* half-life ensures that the compounds remain therapeutically effective in the subject for as long as possible before being metabolised or excreted by the body, which permits administration of lower effective dosages and/or less frequent dosing of the therapeutic compounds.

Accordingly, such compounds, with increased half-life are of significant pharmaceutical importance. In view of the above, there is an unmet need for new compounds with an improved *in-vivo* half life that may be used in the treatment and prevention of medical conditions in which modulation of sortilin is beneficial, such as a neurodegenerative disorder, an inflammatory disorder, a lysosomal storage disorder, a cancer, pain, diabetes mellitus, diabetic retinopathy, glaucoma, uveitis, cardiovascular disease, hereditary eye conditions or hearing loss. The neurodegenerative disorder may be selected from frontotemporal dementia, Alzheimer's disease, Parkinson's disease and spinal cord injury; the inflammatory disorder may be selected from inflammatory diseases and neuroinflammation; the cancer may be selected from breast cancer, lung cancer, ovarian cancer, prostate cancer, thyroid cancer, pancreatic cancer, glioblastoma and colorectal cancer; and the hearing loss may be selected from noise-induced hearing loss, ototoxicity induced hearing loss, age-induced hearing loss, idiopathic hearing loss, tinnitus and sudden hearing loss.

### DISCLOSURE OF THE INVENTION

In a first aspect, the present invention provides a compound of formula (I) 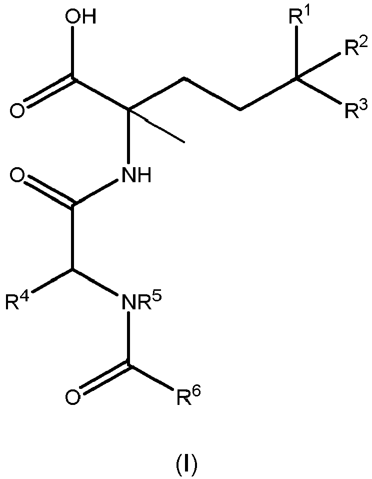
R¹, R² and R³ are each independently selected from the group consisting of halo, H, (C₁-C₄)alkyl, halo-(C₁-C₄)alkyl, (C₂-C₄)alkenyl, and halo-(C₂-C₄)alkenyl;
R⁴ is selected from the group consisting of H, (C₁-C₁₀)alkyl, halo-(C₁-C₁₀)alkyl, (C₂-C₁₀)alkenyl, halo-(C₂-C₁₀)alkenyl, (C₃-C₂₀)aryl, halo-(C₃-C₂₀)aryl, (C₃-C₈)heteroaryl, halo-(C₃-C₂₀)heteroaryl, (C₁-C₆)-alkylene-(C₃-C₂₀)-aryl, (C₁-C₆)-alkylene-(C₃-C₂₀)-heteroaryl, (C₁-C₆)-alkylene-(3- to 10- membered-heterocyclic ring);
   wherein the aryl group in (C₁-C₆)-alkylene-(C₃-C₂₀)-aryl, the heteroaryl group in (C₁-C₆)-alkylene-(C₃-C₂₀)-heteroaryl or the heterocyclic ring in (C₁-C₆)-alkylene-(3- to 10- membered heterocyclic ring) is optionally substituted with one or more substituents independently selected from halo, H, -OH, (C₁-C₄)alkyl, halo-(C₁-C₄)alkyl, (C₁-C₄)alkoxy and halo-(C₁-C₄)alkoxy;
R⁵ is selected from the group consisting of H, (C₁-C₄)alkyl, halo-(C₁-C₄)alkyl, (C₂-C₄)alkenyl and halo-(C₁-C₄)alkenyl;
wherein R⁶ is selected from:
   (a) the group consisting of (C₁-C₆)alkyl, halo-(C₁-C₆)alkyl, (C₂-C₆)alkenyl, halo-(C₂-C₆)alkenyl, (C₃-C₂₀)aryl, halo-(C₃-C₂₀)aryl, (C₃-C₂₀)heteroaryl and halo-(C₃-C₂₀)heteroaryl; or
   (b) 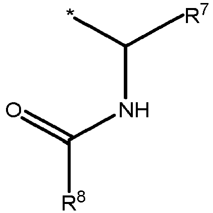
wherein R⁷ is selected from the group consisting of H, (C₁-C₁₀)alkyl, and (C₂-C₁₀)alkenyl;
   wherein the alkyl and alkenyl groups are optionally substituted with halo, an amide group and phenol; and
R⁸ is selected from the group consisting of (C₁-C₁₀)alkyl, halo-(C₁-C₁₀)alkyl, (C₂-C₁₀)alkenyl, halo-(C₂-C₁₀)alkenyl and 3- to 10- membered-heterocyclic ring;
   wherein the heterocyclic ring is optionally substituted with one or more substituents independently selected from halo, -OH, (C₁-C₄)alkyl, halo-(C₁-C₁₀)alkyl, acetyl, (C₁-C₄)alkoxy, and halo-(C₁-C₄)alkoxy.

It is expected that that compounds of formula (I) inhibit or antagonise sortilin and therefore may be useful in conditions where sortilin inhibition is beneficial. Such conditions include a neurodegenerative disorder, an inflammatory disorder, a lysosomal storage disorder, a cancer, pain, diabetes mellitus, diabetic retinopathy, glaucoma, uveitis, cardiovascular disease, hereditary eye conditions or hearing loss. The neurodegenerative disorder may be selected from frontotemporal dementia, Alzheimer's disease, Parkinson's disease and spinal cord injury; the inflammatory disorder may be selected from inflammatory diseases and neuroinflammation; the cancer may be selected from breast cancer, lung cancer, ovarian cancer, prostate cancer, thyroid cancer, pancreatic cancer, glioblastoma and colorectal cancer; and the hearing loss may be selected from noise-induced hearing loss, ototoxicity induced hearing loss, age-induced hearing loss, idiopathic hearing loss, tinnitus and sudden hearing loss.

It is expected that the compounds of the invention may exhibit a longer *in-vivo* half life than the compounds disclosed in European Application No. EP21170274.1 and its corresponding PCT application No. PCT/EP2022/060742; and European Application No. EP21173972.7 and its corresponding PCT Application No. PCT/EP2022/063049, the entire contents of which are herein incorporated by reference. The difference between the compounds of the invention and the compounds disclosed in these earlier applications, is the presence of the additional methyl group on the backbone of formula (I), which provides a further quaternary carbon centre.

Without wishing to be bound by theory, it is believed that unlike the backbone of the previously claimed compounds, the amino acid backbone of formula (I) of the invention, i.e. the following residue: 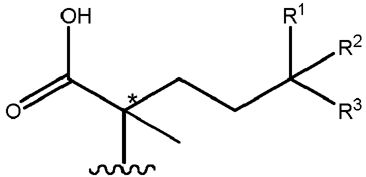 is not recognised by peptidases as a natural amino acid residue due to the additional methyl group on the backbone to form a further quaternary carbon centre at the position denoted *. Accordingly, it is expected that the unnatural amino acid residue above will resist degradation for longer, will metabolise at a slower rate, and will therefore exhibit an increased *in-vivo* half life. It is expected that these effects will be achieved without having a material impact on the ability of the compound to bind to sortilin.

It is therefore expected that the compounds of formula (I) may be dosed at lower concentrations and/or at a lesser frequency than the compounds disclosed in European Application No. EP21170274.1 and its corresponding PCT application No. PCT/EP2022/060742; and European Application No. EP21173972.7 and its corresponding PCT Application No. PCT/EP2022/063049.

It has been demonstrated in European Application No. EP21170274.1 and its corresponding PCT application No. PCT/EP2022/060742; and European Application No. EP21173972.7 and its corresponding PCT Application No. PCT/EP2022/063049 that the compounds of formula (I) without the additional methyl group at the quaternary carbon centre denoted * effectively bind to sortilin and modulate its activity. Accordingly, it is expected that the compounds of the present invention with the additional methyl group will also exhibit similar binding.As used herein, the term "sortilin" may refer to full length sortilin (also referred to as immature sortilin), comprising a signal peptide, a propeptide, a Vps10p domain, a 10CC domain, a transmembrane domain and a large cytoplasmic tail, having an amino acid sequence according to SEQ ID NO: 1 or SEQ ID NO: 2, or it may refer to mature sortilin, comprising a Vps10p domain, a 10CC domain, a transmembrane domain and a large cytoplasmic tail, having an amino acid sequence according to SEQ ID NO: 3, or a naturally occurring fragment, homologue or variant thereof. The term "sortilin" or "sortilin molecule" are used interchangeably herein. It is understood that sortilin is capable of interacting with a pro-neurotrophin molecule to form a sortilin/pro-neurotrophin complex. This sortilin/pro-neurotrophin complex may or may not be capable of interacting with a p75NTR molecule to form a trimeric complex comprising sortilin, pro-neurotrophin and p75NTR. It is understood that this trimeric complex may be responsible for adverse biological responses, such as stimulating apoptosis in retinal and ganglion cells, and controlling growth cone retraction of projecting axons.^{5, 7, 15, 16}

As used herein, the term "pro-neurotrophin" refers to the larger precursors of neurotrophins, which undergo proteolytic cleavage to yield the mature form of the neurotrophin. Neurotrophins are a family of proteins that induce the survival, development and function of neurons, and are commonly referred to as growth factors. Pro-neurotrophins are biologically active and have distinct roles compared to their neurotrophin counterparts, such as induction of apoptosis. Examples of pro-neurotrophins include proNGF, proBDNF, proNT3 and proNT4. Pro-neurotrophins may also control synaptic plasticity. Whereas mature neurotrophins induce synaptic strength, in their proforms they may weaken synapses,

The compounds of the invention may be sortilin inhibitors or antagonists. As used herein, the term "sortilin antagonist" refers to a substance that interferes with, blocks, or otherwise attenuates the effect of, a sortilin protein binding to a pro-neurotrophin (e.g., proNGF, proNT3, proBDNF) and preventing the formation of the trimeric complex between sortilin, p75NTR and the pro-neurotrophin. The term "sortilin antagonist" also includes a substance or agent that interferes with the formation of a high affinity trimeric complex. In the latter scenario, it is recognised that a trimeric complex may be formed in that sortilin can bind to p75NTR (but not proNGF) and p75NTR can simultaneously bind the NGF domain of proNGF. However, the resulting trimeric complex may be of lower affinity for its receptor and as a result have significantly reduced capacity to stimulate apoptosis via the mechanism described above. Skeldal et al (J. Biol. Chem. 2012 Dec 21;287(52):43798-809)¹⁷ demonstrated that the apoptotic function of the trimeric complex is abolished when sortilin is devoid in its intracellular domain. The term "sortilin antagonist" also includes a substance or agent that interferes with, blocks, or otherwise attenuates the effect of, a sortilin protein interacting with p75NTR. This interaction may be completely prevented, in which case the trimeric complex is prevented from forming, or only partially prevented, in which case the trimeric complex may be formed but may have reduced biological potency. Skeldal *et al* showed that complex formation between sortilin and p75NTR relies on contact points in the extracellular domains of the receptors and that the interaction critically depends on an extracellular juxtamembrane 23-amino acid sequence of p75NTR. Thus, the sortilin antagonist may interfere with this 23-amino acid sequence or proximal sequences in the molecules.

It is preferred that R¹, R² and R³ are each independently selected from the group consisting of halo, (C₁-C₂)alkyl and halo-(C₁-C₂)alkyl.

In a preferred aspect of the invention, R¹, R² and R³ are each independently selected from F, CH₃ and CF₃. Most preferably, R¹, R² and R³ are the same. For example, in an exemplary compound of the invention, R¹, R² and R³ may each be F, CH₃ or CF₃.

In another preferred aspect of the invention, R⁴ is selected from the group consisting of H, (C₁-C₆)alkyl, halo-(C₁-C₆)alkyl, (C₃-C₁₀)aryl, (C₁-C₃)-alkylene-(C₃-C₁₀)-aryl, (C₁-C₃)-alkylene-(C₃-C₂₀)-heteroaryl and (C₁-C₃)-alkylene-(3- to 10-membered-heterocyclic ring). The aryl group in (C₁-C₃)-alkylene-(C₃-C₁₀)-aryl, the heteroaryl group in (C₁-C₃)-alkylene-(C₃-C₁₀)-heteroaryl or the heterocyclic ring in (C₁-C₃)-alkylene-(3- to 10- membered heterocyclic ring) is optionally substituted with one or more substituents independently selected from halo, H, -OH, (C₁-C₄)alkyl, halo-(C₁-C₄)alkyl, (C₁-C₄)alkoxy and halo-(C₁-C₄)alkoxy.

The alkyl, haloalkyl, alkenyl, haloalkenyl groups and the alkyl, haloalkyl, alkoxy and haloalkoxy substituents may be linear or branched.

The substituent may be attached at any position of the aryl, heteroaryl or heterocyclic ring. The one or more substituents may be attached to a carbon atom, heteroatom or combinations thereof. Preferably, there are no substituents or between one to three substituents.

The heteroaryl or heterocyclic ring may comprise one, two or more heteroatoms. Preferably, the heteroaryl or heterocyclic ring comprises one or two heteroatoms. The heteroatom may be selected from N, S or O. In groups with more than one heteroatom present, the heteroatoms may be the same or they may be different.

The heterocyclic ring may be aliphatic. It may be monocyclic, bicyclic or tricyclic. Preferably, the heterocyclic ring is monocyclic or bicyclic. Preferably, the heterocyclic ring has between 5-10 members, more preferably between 5-9 members.

The aryl and heteroaryl groups may also be monocyclic, bicyclic or tricyclic. Preferably, monocyclic or bicyclic. Preferably, the aryl and heteroaryl groups have a ring size of between 5-10 members, more preferably between 5-9 members.

In some preferred embodiments, R⁶ is (a) and R⁴ is selected from the group consisting of:

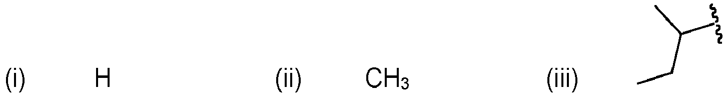

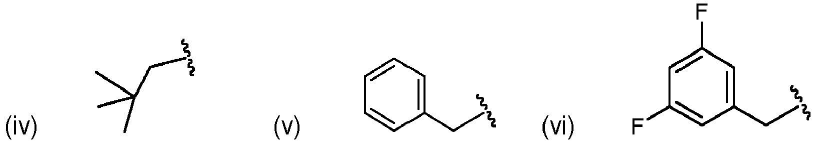

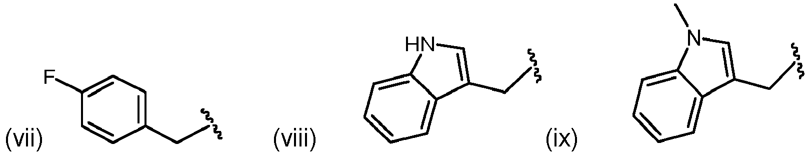

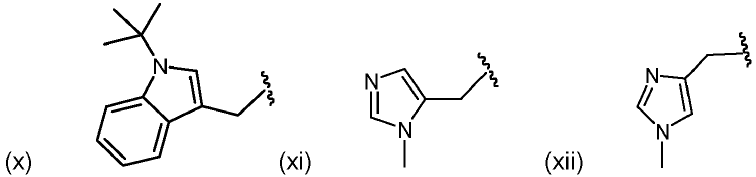

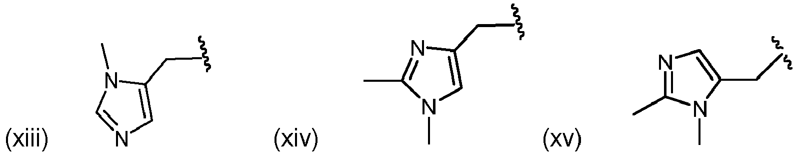

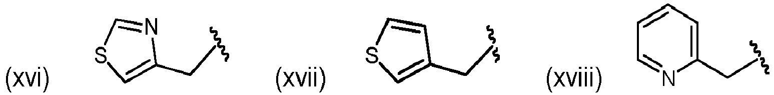

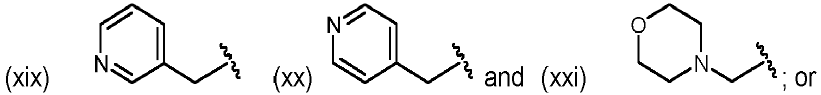

R⁶ is (b) and R⁴ is selected from:

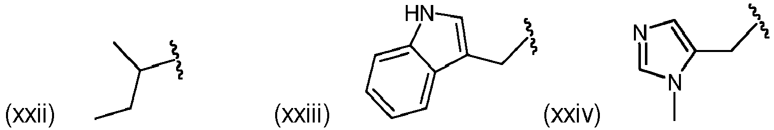

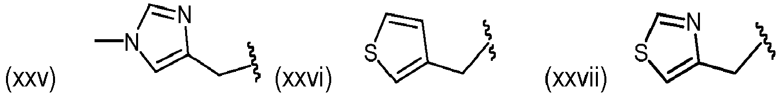

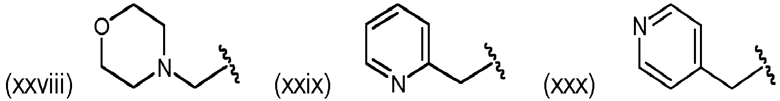

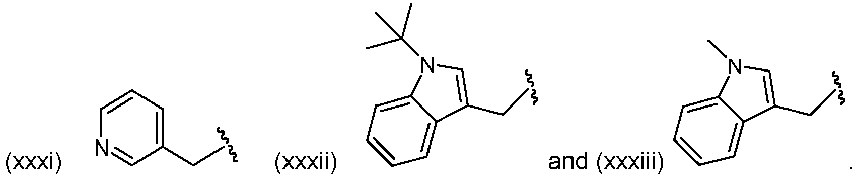

The chirality of the carbon of formula (I) attached to R⁴ is either (R) or (S). Preferably, when groups (ii)-(xii) are included in formula (I), the chirality of this carbon is (S); and when group (i) is included in formula (I), the chirality of this carbon is (S) or (R).

In preferred embodiments, R⁵ is selected from the group consisting of H, (C₁-C₃)-alkyl and (C₁-C₃)haloalkyl. For example, R⁵ may be selected from the group consisting of H and CH₃. Preferably, R⁵ is H. In particular, when R⁶ is (b), preferably R⁵ is H.

When R⁶ is (b), R⁷ is preferably selected from the group consisting of H and (C₁-C₄)alkyl. The alkyl group is optionally substituted with halo, an amide group and phenol.

Particularly preferred R⁷ groups include those selected from the group consisting of:

(i) H

(ii) CH₃

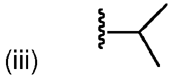

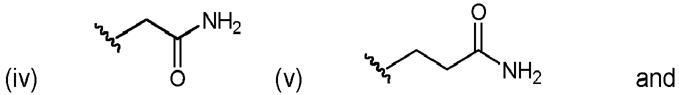

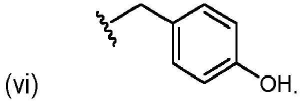

When R⁶ is (b), R⁸ is preferably selected from the group consisting of (C₁-C₃)alkyl, halo-(C₁-C₃)alkyl, and 3- to 8- membered-heterocyclic ring. The heterocyclic ring is optionally substituted with one or more substituents independently selected from halo, -OH, (C₁-C₄)alkyl and acetyl.

Particularly preferred R⁸ groups include those selected from the group consisting of:

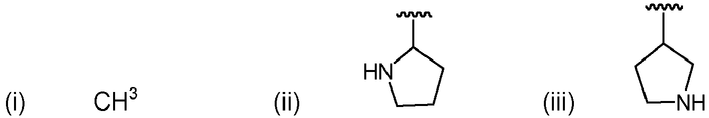

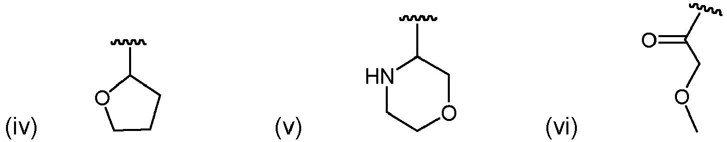

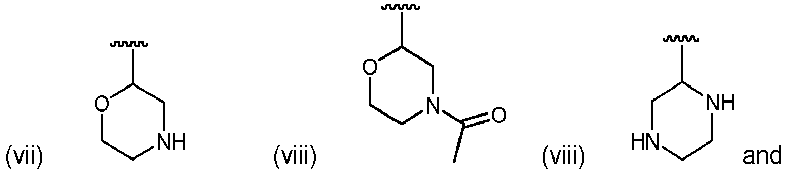

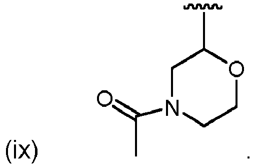

When R⁶ is (a), preferably it is selected from the group consisting of (C₁-C₄)alkyl, halo-(C₁-C₄)alkyl, (C₃-C₈)heteroaryl, and halo-(C₃-C₈)heteroaryl. More preferably, R⁶ is selected from the group consisting of CH₃, pyrazine and morpholine.

Particular compounds of the invention are those listed below.

| **Compound Number** | **Structure** | **IUPAC Name** |
|---|---|---|
| 1 | 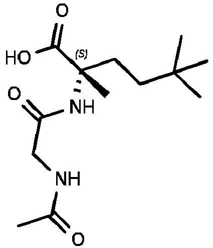 | (2S)-2-(2-acetamidoacetamido)-2,5,5-trimethylhexanoic acid |
| 2 | 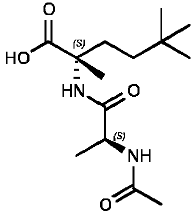 | (2S)-2-[(2S)-2-acetamidopropanamido]-2,5,5-trimethylhexanoic acid |
| 3 | 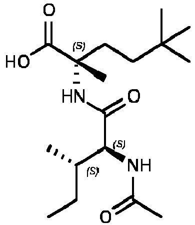 | (2S)-2-[(2S,3S)-2-acetamido-3-methylpentanamido]-2,5,5-trimethylhexanoic acid |
| 4 | 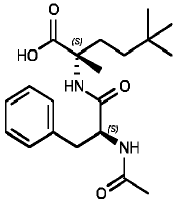 | (2S)-2-[(2S)-2-acetamido-3-phenylpropanamido]-2,5,5-trimethylhexanoic acid |

| | | |
|---|---|---|
| 5 | 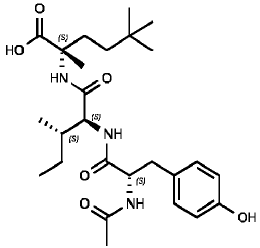 | (2S)-2-[(2S,3S)-2-[(2S)-2-acetamido-3-(4-hydroxyphenyl)propanamido]-3-methylpentanamido]-2,5,5-trimethylhexanoic acid |

The compounds of formula (I) of the invention are intended for use in the treatment or prevention of a neurodegenerative disorder, an inflammatory disorder, a lysosomal storage disorder, a cancer, pain, diabetes mellitus, diabetic retinopathy, glaucoma, uveitis, cardiovascular disease, hereditary eye conditions or hearing loss.

Preferably the neurodegenerative disorder is selected from frontotemporal dementia, Alzheimer's disease, Parkinson's disease and spinal cord injury.

Preferably the inflammatory disorder may be selected from inflammatory diseases and neuroinflammation;
Preferably, the lysosomal storage disorder is selected from the group consisting of NCL/Batten Disease caused by mutations in CLN gene *CLN1 (PPT1), CLN2 (TPP1), CLN3, CLN4 (DNAJC5), CLN5, CLN6, CLN7 (MFSD8), CLN8, CLN10 (CTSD), CLN11, CLN12 (ATP13A2), CLN13 (CTSF), CLN14 (KCTD7), CLCN6,* and/or SGSH; Pompe disease, Fabry disease, Gaucher disease, Niemann-Pick disease Types A, B, and C; GM1 gangliosidosis, GM2 gangliosidosis (including Sandhoff and Tay-Sachs), mucopolysachariddoses (MPS) types I (Hurler disease)/II (Hunter disease)/llla (Sanfilippo A)/IIIB (Sanfilippo B)/lllc (Sanfilippo C)/Illd (Sanfilippo D)/IVA (Morquio A)/IVBNI/VII (Sly)/IX, mucolipisosis III (I-cell) and IV, multiple sulfatase deficiency; sialidosis, galactosialidosis, α-mannosidosis, β-mannosidosis, apartylglucosaminuria, fucosidosis, Schindler disease, metachromatic leukodystrophy caused by deficiencies in either arylsulfatase A or Saposin B, globoid cell leukodystrophy (Krabbe disease), Farber lipogranulomatosis, Wolman and cholesteryl ester storage disease, pycnodystostosis, cystinosis, Salla disease, Oanon disease, Griscelli disease Types 1/2/3, Hermansky Pudliak Disease, and Chédiak-Higashi syndrome.

Preferably the cancer is selected from breast cancer, lung cancer, ovarian cancer, prostate cancer, thyroid cancer, pancreatic cancer, glioblastoma and colorectal cancer.

Preferably the hearing loss is selected from noise-induced hearing loss, ototoxicity induced hearing loss, age-induced hearing loss, idiopathic hearing loss, tinnitus and sudden hearing loss.

Thus, in an embodiment, the compounds for use according to the invention may disrupt interaction between a sortilin molecule and a pro-neurotrophin molecule, or disrupt the interaction between a sortilin molecule and a p75NTR molecule. Said sortilin molecule may be mature sortilin.

Preferably, the compounds of the present invention are sortilin inhibitors. As used herein, the term "sortilin inhibitor" refers to a compound that binds to a sortilin protein, thereby preventing it from binding to a pro-neurotrophin or a p75NTR molecule and preventing the formation of the aforementioned trimeric complex, or resulting in the formation of a trimeric complex that is less active or inactive.

Preferably, the compounds of the present invention prevent the protein-protein interaction between a sortilin molecule and a pro-neurotrophin or a p75NTR molecule, further preventing the formation of the apoptotic trimeric complex usually formed between sortilin, pro-neurotrophin and the p75NTR receptor, or resulting in the formation of a low affinity trimeric complex, which is biologically less active or inactive or has minimal activity.

Thus, the compound may bind to sortilin, a pro-neutrophin or a p75NTR molecule. The antagonistic action may be due to direct blocking of protein-protein interaction or it could be by steric hindrance when bound at a site of one of these proteins apart from the binding site.

It is envisioned that the compounds of the invention will have improved resistance to proteolytic degradation as compared with equivalent compounds without the methyl group in the backbone of formula (I), and that such compounds will have improved pharmacokinetic properties *in vivo.*

According to a second aspect of the invention, there is provided a pharmaceutical composition comprising a compound according to the first aspect of the invention and one or more pharmaceutically acceptable carriers, excipients, and/or diluents.

According to a third aspect of the invention, there is provided a pharmaceutical composition comprising a compound according to the first aspect of the invention and one or more of:
(i) one or more pharmaceutically acceptable carriers, excipients, and/or diluents;
(ii) a ρ75 neurotrophin receptor (NGFR) modulator;
(iii) a glucagon-like peptide-1 receptor (GLP-1R) agonist; and/or
(iv) a nucleic acid encoding a gene therapy expression product capable of substituting for a protein deficient in a lysosomal storage disorder and/or neurological disorder.

Without wishing to be bound by theory, it is believed the pharmaceutical compositions according to the third aspect of the invention are especially efficacious in the treatment of lysosome storage disorders.

Preferably, the NGFR modulator comprises a compound of the formula (II): 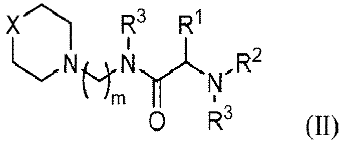
or pharmaceutically acceptable salts thereof, wherein m is an integer in a range of 1 to 8;
X is 0 or NR, where R is hydrogen or C₁-C₆ alkyl;
R¹ is hydrogen, halogen, C₁-C₈ alky I optionally substituted with one or more R⁴, C₁-C₆ haloalkyl, -OH, -NH₂, -NH(C₁-C₆ alkyl), -N(C₁-C₆ alkyl)₂, C₁-C₆ alkoxy, C₁-C₆ haloalkoxy, cycloalkyl(C₀-C₆ alkyl)-optionally substituted with one or more R⁵, or aryl(C₀-C₆ alkyl)- optionally substituted with one or more R⁵;
R² is hydrogen, C₁-C₆ alkyl optionally substituted with one or more R⁴, cycloalkyl(C₀-C₆ alkyl)- optionally substituted with one or more R⁵ aryl(C₀-C₆ alkyl)-optionally substituted with one or more R⁵ or heteroaryl(C₀-C₆ alkyl)- optionally substituted with one or more R⁵; and
each R³ is independently hydrogen or C₁ -C₆ alkyl; wherein each R⁴ is independently selected from the group consisting of -NO₂, -CN, -OH, C₁-C₆ alkoxy, and C₁-C₆ haloalkoxy; and each R⁵ is independently selected from the group consisting of halogen, -NO₂, -CN, C₁ -C₆ alkyl, C₁-C₆ haloalkyl, -OH, C₁-C₆ alkoxy, and C₁-C₆ haloalkoxy.

Preferably, the GLP-1R agonist is selected from the group consisting of semaglutide, exenatide, liraglutide, lixisenatide, albiglutide, dulaglutide, taspoglutide, compounds that inhibit dipeptidyl peptidase-4 (DDP-4, which degrades GLP-1) including but not limited to sitaghptin, vildagliptin, saxagliptin, linagliptin, gemigliptin, anagliptin, teneligliptin, alogliptin, trelagliptin, omarigliptin, evogliptin, gosogliptin, dutogliptin, and berberine; compounds that elicit GLP-1 secretion including metformin; GLP-1R agonist peptides, or nucleic acid constructs encoding such agonist peptides, including but not limited to peptides consisting of at least eight contiguous amino acids of GLP-1R ligands such as glucagon-like peptide-1 (GLP-1) and exendin-4, or mutated versions thereof; anti-GLP-1R agonist antibodies or antibody fragments; combinations thereof; or pharmaceutically acceptable salts thereof.

Preferably, the gene therapy expression products encode proteins implicated in lysosomal storage disorders. In one embodiment, the gene therapy expression product encodes PPT1, TPP1, CLN3, CLN4 (DNAJC5), CLN5, CLN6, CLN7 (MFSD8), CLN8, CLN10 (CTSD), CLN11, CLN12 (ATP13A2), CLN13 (CTSF), CLN14 (KCTD7) or functional fragment thereof. In another embodiment, the gene therapy expression product encodes lysosomal alpha-glucosidase (GAA), alpha-galactosidase A (GLA), glucosylceramidase beta (GBA), acid sphingomyelinase (SMPD1), NPC intracellular cholesterol transporter 1 (NPC1), NPC intracellular cholesterol transporter 2 (NPC2), beta-galactosidase 1 (GLB1), beta-hexosamidase A (HEXA), GM2 ganglioside activator (GM2A), alpha-L iduronidase (IDUA), iduronate 2-sulfatase (IDS), N-sulfoglucosamine sulfohydrolase (SGSH), N- acetylglucosaminidase (NAGLU), heparan-alpha-glucosaminide N-acetyltransferase (HGSNAT), N-acetylglucosamine-6-sulfalase (GNS), N-acetylgalactosamine-6-sulfatase (GALNS), arylsulfatase B (ARSB), beta-glucoronidase (GUSB), hyaluronidase 1 (HYAL1), N-acetylglucosamine-1-phosphate transferase subunits alpha and beta (GNPTAB), mucolipin 1 (MCOLN1), sulfatase modifying factor 1 (SUMF1), neuraminidase 1 (NEU 1), cathepsin A (CTSA), alpha-mannosidase (MAN2B1), beta-mannosidase (MANBA), aspartylglucosaminidase (AGA), alpha-L-fusocidase (FUCA1), alpha-N-acetylgalactosaminidase (NAGA), arylsulfatase A (AREA), prosaposin (PSAP), galactosylceramidase (GALC), acid ceramidase 1 (ASAH1), lipase A (LAI), cathepsin K (CTSK), cystinosin (CTNS), solute carrier family 17 member 5 (SLC17A5), lysosomal associated membrane protein-2 (LAMP2), myosin VA (MY05A), Rab27a member RAS oncogene family (RAB27A), melanophilin (MLPH), biogenesis of lysosomal organelles complex 3 subunit 1 (HPS1), AP-2 complex subunit beta-1 (AP3B1), biogenesis of lysosomal organelles complex 2 subunit 1 (HPS3), biogenesis of lysosomal organelles complex 3 subunit 2 (HPS4), biogenesis of lysosomal organelles complex 2 subunit 2 (HPS5), biogenesis of lysosomal organelles complex 2 subunit 3 (HPS6), dystrobrevin binding protein 1 (OTNBPI), biogenesis of lysosomal organelles complex 1 subunit 3 (BLOC1S3, PLDN) adaptor related protein complex 3 subunit delta 1 (AP3D1), lysosomal trafficking regulator (LYST), or functional fragments thereof.

In a fourth aspect of the invention, there is provided a compound according to the first aspect of the invention, or a pharmaceutical composition according to the second aspect of the invention, or a pharmaceutical composition according to the third aspect of the invention for use in therapy.

According to a fifth aspect of the invention, there is provided a compound according to the first aspect of the invention, or a pharmaceutical composition according to the second aspect of the invention, or a pharmaceutical composition according to the third aspect of the invention for use in the treatment or prevention of a neurodegenerative disorder, an inflammatory disorder, a lysosomal storage disorder, a cancer, pain, diabetes mellitus, diabetic retinopathy, glaucoma, uveitis, cardiovascular disease, hereditary eye conditions or hearing loss.

Preferably, the neurodegenerative disorder is selected from frontotemporal dementia, Alzheimer's disease, Parkinson's disease and spinal cord injury.

Preferably, the hearing loss is selected from noise-induced hearing loss, ototoxicity induced hearing loss, age-induced hearing loss, idiopathic hearing loss, tinnitus and sudden hearing loss.

Preferably, the cancer is selected from breast cancer, lung cancer, ovarian cancer, prostate cancer, thyroid cancer, pancreatic cancer, glioblastoma, and colorectal cancer.

Preferably the cardiovascular disease is selected from atherosclerosis, cardiomyopathy, heard attack, arrhythmias, and coronary artery disease.

According to a sixth aspect of the invention, there is provided the use of the compound according to the first aspect of the invention for the manufacture of a medicament for the treatment or prevention of a neurodegenerative disorder, an inflammatory disorder, a lysosomal storage disorder, a cancer, pain, diabetes mellitus, diabetic retinopathy, glaucoma, uveitis, cardiovascular disease, hereditary eye conditions or hearing loss.

According to a seventh aspect of the invention, there is provided a method for the treatment or prevention of a disease or condition responsive to sortilin modulation comprising administering a therapeutically effective amount of the compound according to the first aspect of the invention or the pharmaceutical composition according the second aspect of the invention, or the pharmaceutical composition according the third aspect of the invention.

The compounds of the invention may include isotopically-labelled and/or isotopically-enriched forms of the compounds. The compounds of the invention herein may contain unnatural proportions of atomic isotopes at one or more of the atoms that constitute such compounds. Examples of isotopes that can be incorporated into the disclosed compounds include isotopes of hydrogen, carbon, nitrogen, oxygen, phosphorus, sulfur, chlorine, such as ²H, ³H, ¹¹C, ¹³C, ¹⁴C, ¹³N, ¹⁵O, ¹⁷O, ³²P, ³⁵S, ¹⁸F, ³⁶Cl.

The compounds of the invention may be used as such or, where appropriate, as pharmacologically acceptable salts (acid or base addition salts) thereof. The pharmacologically acceptable addition salts mentioned below are meant to comprise the therapeutically active non-toxic acid and base addition salt forms that the compounds are able to form. Compounds that have basic properties can be converted to their pharmaceutically acceptable acid addition salts by treating the base form with an appropriate acid. Exemplary acids include inorganic acids, such as hydrogen chloride, hydrogen bromide, hydrogen iodide, sulphuric acid, phosphoric acid; and organic acids such as formic acid, acetic acid, propanoic acid, hydroxyacetic acid, lactic acid, pyruvic acid, glycolic acid, maleic acid, malonic acid, oxalic acid, benzenesulphonic acid, toluenesulphonic acid, methanesulphonic acid, trifluoroacetic acid, fumaric acid, succinic acid, malic acid, tartaric acid, citric acid, salicylic acid, p-aminosalicylic acid, pamoic acid, benzoic acid, ascorbic acid and the like. Compounds that have acidic properties can be converted to their pharmaceutically acceptable basic addition salts by treating the acid form with an appropriate base. Exemplary base addition salt forms are the sodium, potassium, calcium salts, and salts with pharmaceutically acceptable amines such as, for example, ammonia, alkylamines, benzathine, and amino acids, such as, e.g. arginine and lysine. The term addition salt as used herein also comprises solvates which the compounds and salts thereof are able to form, such as, for example, hydrates, alcoholates and the like.

Throughout the present disclosure, a given chemical formula or name shall also encompass all pharmaceutically acceptable salts, solvates, hydrates, N-oxides, and/or prodrug forms thereof. It is to be understood that the compounds of the invention include any and all hydrates and/or solvates of the compound formulas. It is appreciated that certain functional groups, such as the hydroxy, amino, and like groups form complexes and/or coordination compounds with water and/or various solvents, in the various physical forms of the compounds. Accordingly, the above formulas are to be understood to include and represent those various hydrates and/or solvates.

Compounds of the invention also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, amide - imidic acid pairs, enamine - imine pairs, and annular forms where a proton can occupy two or more positions of a heterocyclic system, for example, 1H- and 3H-imidazole, 1H, 2Hand 4H- 1,2,4-triazole, 1H- and 2H- isoindole, and 1H- and 2H-pyrazole. Tautomeric forms can be in equilibrium or sterically locked into one form by appropriate substitution.

The compounds described herein can be asymmetric (e.g. having one or more stereocenters). All stereoisomers, such as enantiomers and diastereomers, are intended unless otherwise indicated. Compounds of the present invention that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present invention. Cis- and trans-geometric isomers of the compounds of the present invention are described and may be isolated as a mixture of isomers or as separated isomeric forms.

In the case of the compounds which contain an asymmetric carbon atom, the invention relates to the D form, the L form, and D,L mixtures and also, where more than one asymmetric carbon atom is present, to the diastereomeric forms. Those compounds of the invention which contain asymmetric carbon atoms, and which as a rule accrue as racemates, can be separated into the optically active isomers in a known manner, for example using an optically active acid. However, it is also possible to use an optically active starting substance from the outset, with a corresponding optically active or diastereomeric compound then being obtained as the end product.

The term "prodrugs" refers to compounds that may be converted under physiological conditions or by solvolysis to a biologically active compound of the invention. A prodrug may be inactive when administered to a subject in need thereof, but is converted in vivo to an active compound of the invention. Prodrugs are typically rapidly transformed in vivo to yield the parent compound of the invention, e.g. by hydrolysis in the blood. The prodrug compound usually offers advantages of solubility, tissue compatibility or delayed release in a mammalian organism (see Silverman, R. B., The Organic Chemistry of Drug Design and Drug Action, 2nd Ed., Elsevier Academic Press (2004), page 498 to 549). Prodrugs of a compound of the invention may be prepared by modifying functional groups, such as a hydroxy, amino or mercapto groups, present in a compound of the invention in such a way that the modifications are cleaved, either in routine manipulation or in vivo, to the parent compound of the invention. Examples of prodrugs include, but are not limited to, acetate, formate and succinate derivatives of hydroxy functional groups or phenyl carbamate derivatives of amino functional groups.

The term "treatment" as used herein may include prophylaxis of the named disorder or condition, or amelioration or elimination of the disorder once it has been established. The term "prevention" refers to prophylaxis of the named disorder or condition.

Methods delineated herein include those wherein the subject is identified as in need of a particular stated treatment. Identifying a subject in need of such treatment can be in the judgment of a subject or a health care professional and can be subjective (e.g. opinion) or objective (e.g. measurable by a test or diagnostic method).

In other aspects, the methods herein include those further comprising monitoring subject response to the treatment administrations. Such monitoring may include periodic imaging or sampling of subject tissue, fluids, specimens, cells, proteins, chemical markers, genetic materials, Etc. as markers or indicators of the treatment regimen. In other methods, the subject is pre-screened or identified as in need of such treatment by assessment for a relevant marker or indicator of suitability for such treatment.

The invention provides a method of monitoring treatment progress. The method includes the step of determining a level of diagnostic marker (Marker) (e.g. any target or cell type delineated herein modulated by a compound herein) or diagnostic measurement (e.g., screen, assay) in a subject suffering from or susceptible to a disorder or symptoms thereof delineated herein, in which the subject has been administered a therapeutic amount of a compound herein sufficient to treat the disease or symptoms thereof. The level of Marker determined in the method can be compared to known levels of Marker in either healthy normal controls or in other afflicted patients to establish the subject's disease status. In preferred embodiments, a second level of Marker in the subject is determined at a time point later than the determination of the first level, and the two levels are compared to monitor the course of disease or the efficacy of the therapy. In certain preferred embodiments, a pre-treatment level of Marker in the subject is determined prior to beginning treatment according to this invention; this pre-treatment level of Marker can then be compared to the level of Marker in the subject after the treatment commences, to determine the efficacy of the treatment.

A level of Marker or Marker activity in a subject may be determined at least once. Comparison of Marker levels, e.g., to another measurement of Marker level obtained previously or subsequently from the same patient, another patient, or a normal subject, may be useful in determining whether therapy according to the invention is having the desired effect, and thereby permitting adjustment of dosage levels as appropriate. Determination of Marker levels may be performed using any suitable sampling/expression assay method known in the art or described herein. Preferably, a tissue or fluid sample is first removed from a subject. Examples of suitable samples include blood, urine, tissue, mouth or cheek cells, and hair samples containing roots. Other suitable samples would be known to the person skilled in the art. Determination of protein levels and/or mRNA levels (e.g., Marker levels) in the sample can be performed using any suitable technique known in the art, including, but not limited to, enzyme immunoassay, ELISA, radiolabeling/assay techniques, blotting/ chemiluminescence methods, real-time PCR, and the like.

For clinical use, the compounds disclosed herein are formulated into pharmaceutical compositions (or formulations) for various modes of administration. It will be appreciated that compounds of the invention may be administered together with a physiologically acceptable carrier, excipient, and/or diluent (i.e. one, two, or all three of these). The pharmaceutical compositions disclosed herein may be administered by any suitable route, preferably by oral, rectal, nasal, topical (including ophthalmic, buccal and sublingual), sublingual, transdermal, intrathecal, transmucosal or parenteral (including subcutaneous, intramuscular, intravenous and intradermal) administration. Other formulations may conveniently be presented in unit dosage form, e.g., tablets and sustained release capsules, and in liposomes, and may be prepared by any methods well known in the art of pharmacy. Pharmaceutical formulations are usually prepared by mixing the active substance, or a pharmaceutically acceptable salt thereof, with conventional pharmaceutically acceptable carriers, diluents or excipients. Examples of excipients are water, gelatin, gum arabicum, lactose, microcrystalline cellulose, starch, sodium starch glycolate, calcium hydrogen phosphate, magnesium stearate, talcum, colloidal silicon dioxide, and the like. Such formulations may also contain other pharmacologically active agents, and conventional additives, such as stabilizers, wetting agents, emulsifiers, flavouring agents, buffers, and the like. Usually, the amount of active compounds is between 0.1-95% by weight of the preparation, preferably between 0.2-20% by weight in preparations for parenteral use and more preferably between 1-50% by weight in preparations for oral administration. The formulations can be further prepared by known methods such as granulation, compression, microencapsulation, spray coating, Etc. The formulations may be prepared by conventional methods in the dosage form of tablets, capsules, granules, powders, syrups, suspensions, suppositories or injections. Liquid formulations may be prepared by dissolving or suspending the active substance in water or other suitable vehicles. Tablets and granules may be coated in a conventional manner. To maintain therapeutically effective plasma concentrations for extended periods of time, compounds disclosed herein may be incorporated into slow release formulations.

The dose level and frequency of dosage of the specific compound will vary depending on a variety of factors including the potency of the specific compound employed, the metabolic stability and length of action of that compound, the patient's age, body weight, general health, sex, diet, mode and time of administration, rate of excretion, drug combination, the severity of the condition to be treated, and the patient undergoing therapy. The daily dosage may, for example, range from about 0.001 mg to about 100 mg per kilo of body weight, administered singly or multiply in doses, e.g. from about 0.01 mg to about 25 mg each. Normally, such a dosage is given orally but parenteral administration may also be chosen.

### DEFINITIONS

"Optional" or "optionally" means that the subsequently described event or circumstance may, but need not, occur, and that the description includes instances where the event or circumstance occurs and instances in which it does not.

The term "heteroatom" means O, N, or S.

The term "(C₁-Cₙ)alkyl" denotes a straight, branched or cyclic or partially cyclic alkyl group having from 1 to n carbon atoms, i.e. 1, 2, 3... or n carbon atoms. For the "(C₁-Cₙ)alkyl" group to comprise a cyclic portion it should be formed of at least three carbon atoms. For parts of the range "(C₁-Cₙ)alkyl" all subgroups thereof are contemplated. For example, in the range (C₁-C₆)alkyl, all subgroups such as (C₁-C₅)alkyl, (C₁-C₄)alkyl, (C₁-C₃)alkyl, (C₁-C₂)alkyl, (C₁)alkyl, (C₂-C₆)alkyl, (C₂-C₅)alkyl, (C₂-C₄)alkyl, (C₂-C₃)alkyl, (C₂)alkyl, (C₃-C₆)alkyl, (C₃-C₅)alkyl, (C₃-C₄)alkyl, (C₃)alkyl, (C₄-C₆)alkyl, (C₄-C₅)alkyl, (C₄)alkyl, (C₅-C₆)alkyl, (C₆)alkyl. Examples of "C₁-C₆ alkyl" include methyl, ethyl, n-propyl, isopropyl, cyclopropyl, n-butyl, isobutyl, sec-butyl, t-butyl, cyclobutyl, cyclopropylmethyl, branched or cyclic or partially cyclic pentyl and hexyl Etc.

The term "halo-(C₁-Cₙ)alkyl" denotes a C₁-Cₙ alkyl as described above substituted with at least one halogen atom, which is preferably, F, Cl, Br and I, more preferably F and CI, and most preferably F.

When a term denotes a range, for instance "1 to 6 carbon atoms" in the definition of (C₁-C₆)alkyl, each integer is considered to be disclosed, i.e. 1, 2, 3, 4, 5 and 6.

The term "(C₂-Cₙ)alkenyl" denotes a straight, branched or cyclic or partially cyclic alkyl group having at least one carbon-carbon double bond, and having from 2 to 6 carbon atoms. The alkenyl group may comprise a ring formed of 3 to 6 carbon atoms. For parts of the range "(C₂-Cₙ)alkenyl" all subgroups thereof are contemplated. For example, the range "(C₂-C₄)alkenyl" covers (C₂-C₄)alkenyl, (C₂-C₃)alkenyl, (C₂)alkenyl. Examples of "(C₂-C₄)alkenyl" include 2-propenyl, 2-butenyl, 3-butenyl, 2-methyl-2-propenyl Etc.

The term "(C₁-C₄)alkoxy" denotes -O-((C₁-C₄)alkyl) in which a (C₁-C₄)alkyl group is as defined above and is attached to the remainder of the compound through an oxygen atom. Examples of "(C₁-C₄)alkoxy" include methoxy, ethoxy, n-propoxy, isopropoxy, n-butoxy, isobutoxy, sec-butoxy and t-butoxy.

The term "halo(C₁-C₄)alkoxy" denotes a (C₁-C₄)alkoxy as described above substituted with a halogen atom, which is preferably, F, Cl, Br and I, more preferably F and CI, and most preferably F.

The term "halo" means a halogen atom, and is preferably, F, Cl, Br and I, more preferably F and CI, and most preferably F.

The term "3- to 10-membered heterocyclic ring" denotes a non-aromatic ring system having 3 to 10 ring atoms, in which at least one ring atoms is a heteroatom.

The term "unnatural amino acid," as used herein, represents amino acid residues that cannot be generated biosynthetically in any organism using unmodified or modified genes from any organism. These include, but are not limited to, modified amino acid residues that are not exhibited in one of the 20 naturally occurring amino acids. Such non-natural amino acid residues can be introduced by substitution of naturally occurring amino acid residues, and/or by insertion of non-natural amino acid residues. The non-natural amino acid residue also can be incorporated such that a desired functionality is imparted to the sortilin modulator, for example, the ability to increase the *in-vivo* half-life of the compounds of formula (I).

The term *"in vivo* half-life" as used herein refers to a biological half-life of a particular compound in the circulation of a given subject and is represented by a time required for half the quantity administered in the subject to be cleared from the circulation and/or other tissues in the subject. The *in vivo* half-life may readily be measured using well-known and routine methods and assays which are known to the skilled person. This is a measure of not only the rate of degradation of the compound by enzymes present in the body (for example in the blood, lymph, saliva, stomach, small and large intestines, liver etc) but also a measure of the level of uptake or sequestration of the compound by or on cells and tissues of the body that may hinder its intended activity or delivery to the site of action, and the rate of excretion of the compound from the body, for example in the urine or via the digestive tract. It is expected that by modifying the compounds to have "non-natural" amino acid groups, the persistence of the compounds in vivo will be increased and therefore the delivery of the compounds to the site of action achieved with lower and less frequent dosing than otherwise required. This is expected to not only have economic benefits in terms of reduced unit pricing but also enable clinical efficacy with reduced potential for side effects in patients.

"An effective amount" refers to an amount of a compound of the invention that confers a therapeutic effect on the treated subject. The therapeutic effect may be objective (i.e. measurable by some test or marker) or subjective (i.e. subject gives an indication of or feels an effect).

As used herein, the terms "administration" or "administering" mean a route of administration for a compound disclosed herein. Exemplary routes of administration include, but are not limited to, oral, intraocular, intravenous, intraperitoneal, intraarterial, and intramuscular. The preferred route of administration can vary depending on various factors, e.g. the components of the pharmaceutical composition comprising a compound disclosed herein, site of the potential or actual disease and severity of disease.

The terms "subject" and "patient" are used herein interchangeably. They refer to a human or another mammal (e.g., mouse, rat, rabbit, dog, cat, cattle, swine, sheep, horse or primate) that can be afflicted with or is susceptible to a disease or disorder but may or may not have the disease or disorder. It is preferred that the subject is human.

As defined herein, a "lysosomal storage disorder" (LSD) is any disorder that is characterized by lysosomal dysfunction and the accumulation of cellular storage material consisting of macromolecular substrates. In some embodiments, the lysosomal storage disorder is an inherited/genetic disorder. A lysosomal storage disorder may be a disorder having a predominantly lysosomal etiology. Lysosomal storage disorders include numerous rare genetic diseases that are most often inherited in a recessive manner. In all of these cases, restoration of lysosomal health and function has the potential to ameliorate a range of cellular and systemic pathologies.

As defined herein, the Neuronal Ceroid Lipofuscinoses (NCLs), also known as Batten Disease, are a group of LSDs characterized by the accumulation of autofluorescent storage material (ASM) in lysosomes, neuroinflammation and neurodegeneration, neurological symptoms and premature death. NCLs are caused by mutations in one of 13 genes and are typically inherited in an autosomal recessive manner.

Compounds of the invention may be disclosed by the name or chemical structure. If a discrepancy exists between the name of a compound and its associated chemical structure, then the chemical structure prevails.

The invention will now be further illustrated by the following non-limiting examples. The specific examples below are to be construed as merely illustrative, and not limitative of the remainder of the disclosure in any way whatsoever. Without further elaboration, it is believed that one skilled in the art can, based on the description herein, utilise the present invention to its fullest extent. All references and publications cited herein are hereby incorporated by reference in their entirety.

### PREPARATION OF COMPOUNDS OF THE INVENTION

The compounds of the invention can be prepared according to the following General Synthetic Schemes by methods well known and appreciated in the art. Suitable reaction conditions, for the steps of these schemes, are well known in the art and appropriate substitutions of solvents and co-reagents are within the common general knowledge of the person skilled in the art. Likewise, it will be appreciated by those skilled in the art that synthetic intermediates may be isolated and/or purified by various well-known techniques as needed or desired, and that frequently, it will be possible to use various intermediates directly in subsequent synthetic steps with little or no purification. Furthermore, the skilled person will appreciate that in some circumstance, the orders in which moieties are introduced is not critical. The particular order of steps required to produce the compounds of formula (I) is dependent upon the particular compound being synthesized, the starting compound, and the relative liability of the substituted moieties as is well appreciated by those of ordinary skill in the art. All substituents, unless otherwise indicated, are as previously defined, and all reagents are well known and appreciated in the art.

### GENERAL SYNTHETIC PROCEDURE 1

The compounds of general formula (I) may be prepared by a variety of procedures, some of which are described below. Compounds of general formula (I), where R⁶ is (a), can be obtained as shown in general Scheme 1 below. The skilled artisan will appreciate that the amine functionality in compounds of general formula Int-1 can be coupled with the free carboxylic acid functionality present in compounds of general formula AA-2, using, as described above and for the purpose of exemplification, a mixture of a coupling reagent and a base, such as HATU and N-methyl morpholine in a suitable solvent system, like DCM, DMF or a mixture thereof, to yield intermediate of general formula Int-2. The chemoselective cleavage of the ester-type protecting group present in compound of general formula Int-2 can then be done to afford the desired compounds of general formula (I), for example by basic hydrolysis in an aqueous media, such as LiOH in a mixture of water and acetonitrile.

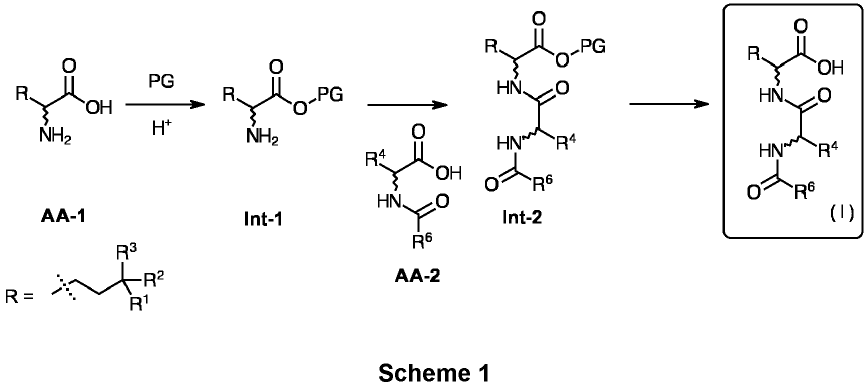

### GENERAL SYNTHETIC PROCEDURE 2

One of the methods particularly suitable for the preparation of small to medium-sized peptides, as is well known and appreciated by those of ordinary skill in the art, is solid phase peptide synthesis (SPPS).

Compounds of general formula (I), where R⁶ is (a), can also be obtained, as shown in general Scheme 2 below. Suitable starting materials and protected amino acids of general formula AA-1, AA-2, Int-1, Int-3 or Int-4 are either commercially available or may be prepared by a variety of methods. For example, as illustrated in Schemes 1 and 2, the carboxylic acid functionality of appropriately substituted amino acids of general formula AA-1, can be chemoselectively protected as a suitable derivative, for example as a methyl ester, using well established procedures and reagents like a mixture of methanol and thionyl chloride to yield a compound of general formula Int-1. In a subsequent step, protection of the free amine functionality in Int-1 as, for example, an amide or a carbamate, like Fmoc, affords and intermediate of general structure Int-3. The intermediate of general formula Int-4 can be obtained by selective deprotection of the masked acid functionality present in Int-3, using for example an acidic hydrolysis. When using SPPS, Int-4 can be attached to a suitably functionalised resin, for example Wang resin, using ester formation reagents like, for exemplification, a mixture of diisopropylmethanediimine, 4-methylmorpholine and N,N-dimethylpyridin-4-amine in dichloromethane, to afford solid supported intermediate of general formula Int-5.

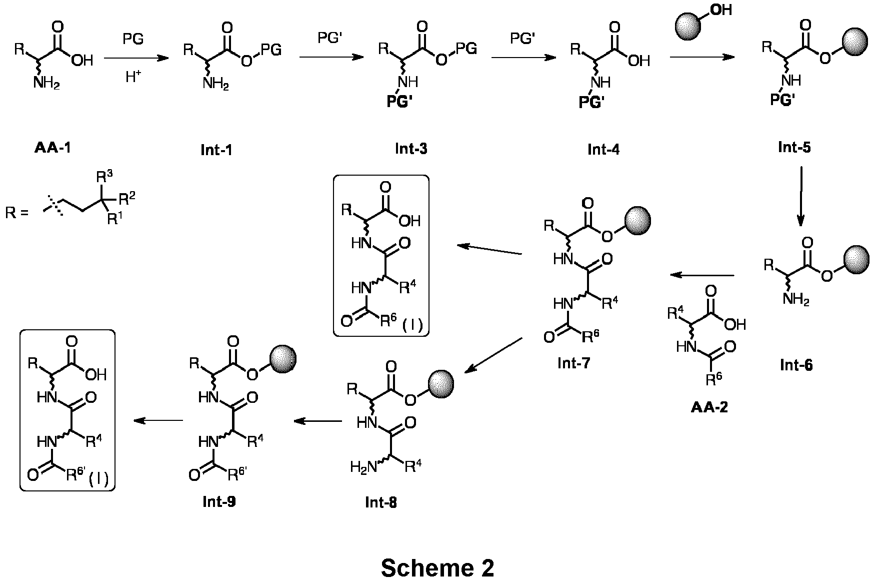

When required the resin bound intermediates Int-5 to Int-8 can be analysed after cleavage from resin. Alternatively, the corresponding steps on solid support can be performed without analysis. The protecting group of the amine in Int-5 can be removed by treatment with a base like piperidine, when, for example, the Fmoc protecting group is used, to yield Int-6. The free acid functionality present in the second N-protected-amino-acid, represented here by the compound of general formula AA-2, can be coupled with the free amine present in intermediate of general formula Int-6 to afford Int-7, using a classical amide coupling procedure, for example a mixture of HATU and a base, like N-methyl morpholine in a suitable solvent system, like a mixture of DCM-DMF. When, advantageously, the N-protecting group in AA-2 and Int-7 has the desired substitution, R⁶, cleavage, for example, by acidic hydrolysis using a reagent like trifluoroacetic acid, of the ester bond between the solid support and the compound of general structure represented in Int-7 yields the desired compound of general formula (I).

Alternatively, AA-2 can be introduced in Int-7 with a suitable protecting group on the amine moiety that can be removed in the subsequent step, to give Int-8, using a base like piperidine, when, for example, the Fmoc protecting group is used. The free basic amine functionality present in Int-8 can be readily functionalized as an acyl derivative using a classical amide coupling procedure, for example a mixture of HATU and a base, such as N-methyl morpholine in a suitable solvent system, like a mixture of DCM-DMF, to yield intermediate of general formula Int-9. The final compound of general formula (I) is obtained, by cleavage, for example, using acidic hydrolysis with a reagent like trifluoroacetic acid, of the ester bond between the resin solid support and the compound of general structure shown in Int-9.

### GENERAL SYNTHETIC PROCEDURE 3

Compounds of general formula (I), where R⁶ is (b), can be obtained, as shown in general Scheme 3 below. Suitable starting materials and protected amino acids of general formula AA-1 Int-1, Int-3 or Int-4 are either commercially available or may be prepared by a variety of methods. For example, as illustrated in **Scheme 3,** the carboxylic acid functionality of appropriately substituted amino acids of general formula AA-1, can be chemoselectively protected as a suitable derivative, for example as a methyl ester, using well established procedures and reagents like a mixture of methanol and thionyl chloride to yield a compound of general formula Int-1. In a subsequent step, protection of the free amine functionality in Int-1 as, for example, an amide or a carbamate, like Fmoc, affords and intermediate of general structure Int-3. The intermediate of general formula Int-4 can be obtained by selective deprotection of the masked acid functionality present in Int-3, using for example an acidic hydrolysis. When using SPPS, Int-4 can be attached to a suitably functionalised resin, for example Wang resin, using ester formation reagents like, for exemplification, a mixture of diisopropylmethanediimine, 4-methylmorpholine and *N*,*N*-dimethylpyridin-4-amine in dichloromethane, to afford solid supported intermediate of general formula Int-5.

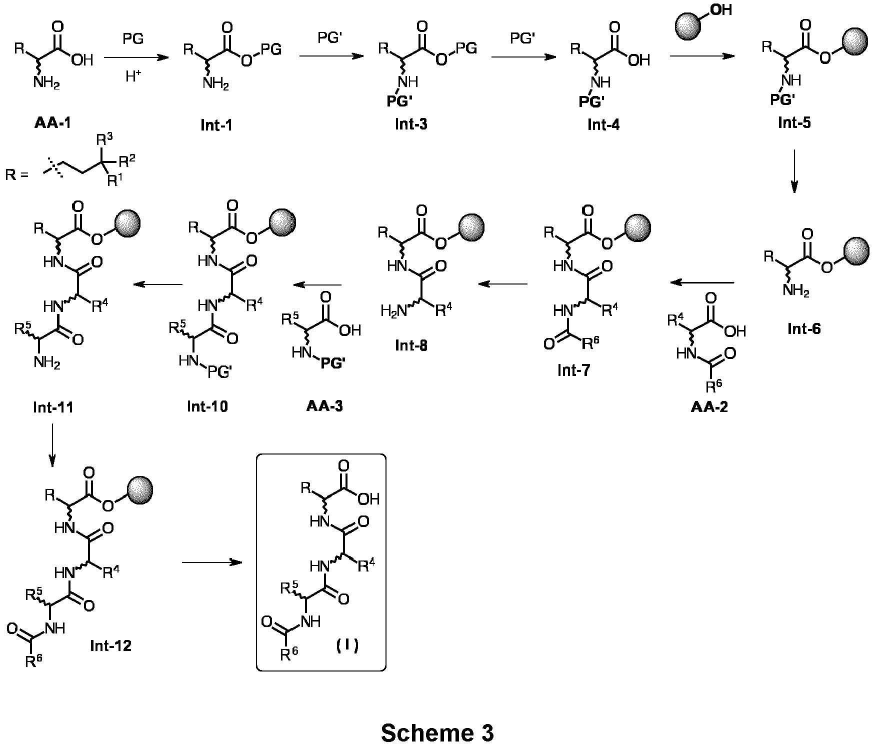

When required the resin bound intermediates Int-5 to Int-11 can be analysed after cleavage from resin. Alternatively, the corresponding steps on solid support can be performed without analysis. The protecting group of the amine in Int-5 can be removed by treatment with a base like piperidine, when, for example, the Fmoc protecting group is used, to yield Int-6. The free acid functionality present in the second *N*-protected-amino-acid, represented here by the compound of general formula AA-2, can be coupled with the free amine present in intermediate of general formula Int-6 to afford Int-7, using a classical amide coupling procedure, for example a mixture of HATU and a base, like *N*-methyl morpholine in a suitable solvent system, like a mixture of DCM-DMF.

Similarly, suitably, amine moiety protected, AA-2 can be coupled with Int-6 to afford Int-7. The temporary amine protecting group, can then be removed in the subsequent step, to give Int-8, using a base like piperidine, when, for example, the Fmoc protecting group is used. The free basic amine functionality present in Int-8 can be, once again and as previously described, extended by introduction, as exemplified in the General Synthetic Procedure, of another suitably protected amino acid derivative of general formula AA-3 to give Int-10. Equally, after selective removal of the protecting group of the amine in Int-10, obtained Int-11 can be readily functionalized as an acyl derivative using a classical amide coupling procedure, for example a mixture of HATU and a base, such as N-methyl morpholine in a suitable solvent system, like a mixture of DCM-DMF, to yield intermediate of general formula Int-12. The final compound of general formula (I), where R⁶ is (b), is obtained, by cleavage, for example, using acidic hydrolysis with a reagent like trifluoroacetic acid, of the ester bond between the resin solid support and the compound of general structure shown in Int-12.

Resin bound intermediates were analysed after cleavage from resin. All the analysis of intermediate are of free acids.

The products of each step can then be recovered by conventional methods including extraction, evaporation, precipitation, chromatography, filtration, trituration, crystallisation and the like.

Resin bound intermediates were analysed after cleavage from resin. All the analysis of intermediate are of free acids.

### GENERAL SYNTHETIC PROCEDURE 4

The compounds of general formula (I) may be prepared by a variety of procedures, some of which are described below. Amino-acids of type AA-1, as shown in the general synthetic **Scheme 4,** can be purchased as racemic mixtures or as single enantiomers. They can be synthesised by methods known to those skilled in the art. Similarly, amide or peptide synthetic steps well known to those versed in the field will afford the compounds of general formula I.

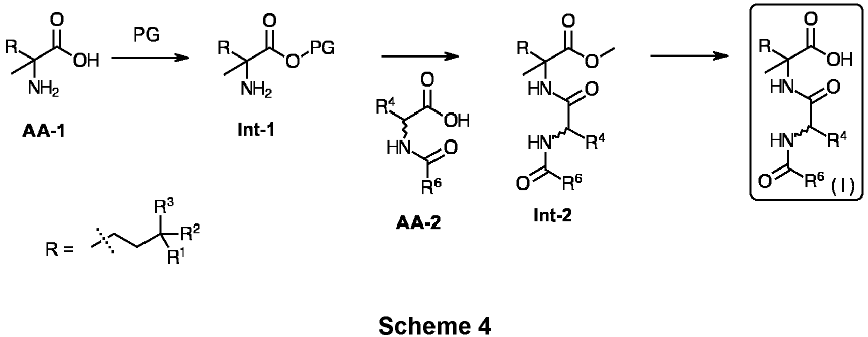

General synthetic **Scheme 5** illustrates the possibility to use solid support strategies towards compounds of general formula I.

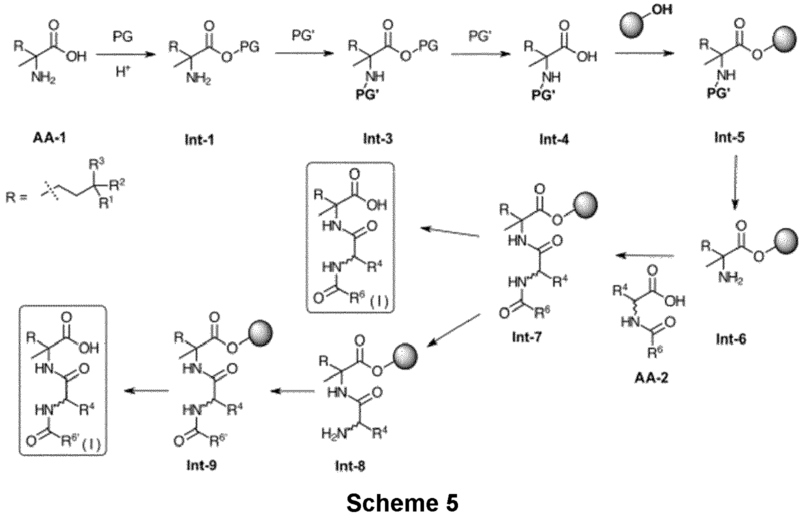

The products of each step can then be recovered by conventional methods including extraction, evaporation, precipitation, chromatography, filtration, trituration, crystallisation, and the like.

Compounds of general formula (I) may contain one or more stereogenic centres. Those can be introduced from available single enantiomers, optically active, starting materials of the type AA-1. The integrity of the existing stereogenic centre can be confirmed by analytical techniques well known to those skilled in the art like for example chiral support high pressure chromatography. Alternatively, when racemic starting materials are used, it will be appreciated that if desired, single isomer products can be obtained as single enantiomers or as single diastereoisomers, by known techniques like preparative chiral support high pressure chromatography.The skilled artisan will also appreciate that not all of the substituents in the compounds of formula (I) will tolerate certain reaction conditions employed to synthesise the compounds. These moieties may be introduced at a convenient point in the synthesis, or may be protected and then deprotected as necessary or desired, as is well known in the art. The skilled artisan will appreciate that the protecting groups may be removed at any convenient point in the synthesis of the compounds of the present invention. Methods for introducing or removing protecting groups used in this invention are well known in the art; see, for example, Greene and Wuts, Protective Groups in Organic Synthesis, 4th Ed., John Wiley and Sons, New York (2006)¹⁶.

### In Vivo Half Life

The *in vivo* half life may be determined by well-known and routine methods and assays which are known to the skilled person. For example, the pharmacokinetic half-life (T_{1/2}) of one of the compounds of the invention can be calculated, after its intra-venous administration to a suitable animal model, by plotting a Plasma Concentration *vs* Time curve on a semi-logarithmic scale. Least square regression analysis is then conducted on the terminal linear part of the curve to calculate the half-life (T_{1/2}).

### EXAMPLES

### Abbreviations

approx: approximately; aq: aqueous; br: broad; *ca.: circa;* CDI: 1,1'-Carbonyldiimidazole; CPME: cyclopentyl methyl ether; d: doublet; DCM: dichloromethane; DIC: *N*,*N'*-Diisopropylcarbodiimide; dioxane: 1,4-dioxane; DIPEA: diisopropylethylamine; DMF: dimethylformamide; eq.: equivalent; Et₃N: triethylamine; EtOAc: ethyl acetate; EtOH: ethanol; Fmoc: fluorenylmethoxycarbonyl; Boc: tert-butoxycarbonyl; h: hours; min: minutes: HATU: 2-(3*H*-[1,2,3]triazolo[4,5-b]pyridin-3-yl)-1,1,3,3-tetramethyl isouronium hexafluorophosphate(V); HPLC: high performance liquid chromatography; IPA, isopropanol; LC: liquid chromatography; m: multiplet; M: molar, molecular ion; MeCN: acetonitrile; MeOH: methanol; MS: mass spectrometry; NMR: nuclear magnetic resonance; PDA: photodiode array; q: quartet; rt: room temperature (*ca*. 20 °C); R_{T}: retention time; s: singlet, solid; SPPS: solid phase peptide synthesis. t: triplet; TBAF: tetrabutylammonium fluoride; TBME: *tert*-butyl methyl ether; TFA: trifluoroacetic acid; THF: tetrahydrofuran; UPLC: ultra-performance liquid chromatography; UV: ultraviolet.

Other abbreviations are intended to convey their generally accepted meaning.

### General Experimental Conditions

All starting materials and solvents were obtained either from commercial sources or prepared according to the literature citation. Reaction mixtures were magnetically stirred, and reactions performed at room temperature (*ca*. 20 °C) unless otherwise indicated.

Column chromatography was performed on an automated flash chromatography system, such as a CombiFlash Rf system, using pre-packed silica (40 µm) cartridges, unless otherwise indicated.

¹H-NMR spectra were recorded at 400 MHz on a Bruker Avance AV-I-400 or on a Bruker Avance AV-II-400 instrument. Chemical shift values are expressed in ppm-values relative to tetramethylsilane unless noted otherwise. The following abbreviations or their combinations are used for multiplicity of NMR signals: br = broad, d = doublet, m = multiplet, q = quartet, quint = quintet, s = singlet and t = triplet.

### Example 1

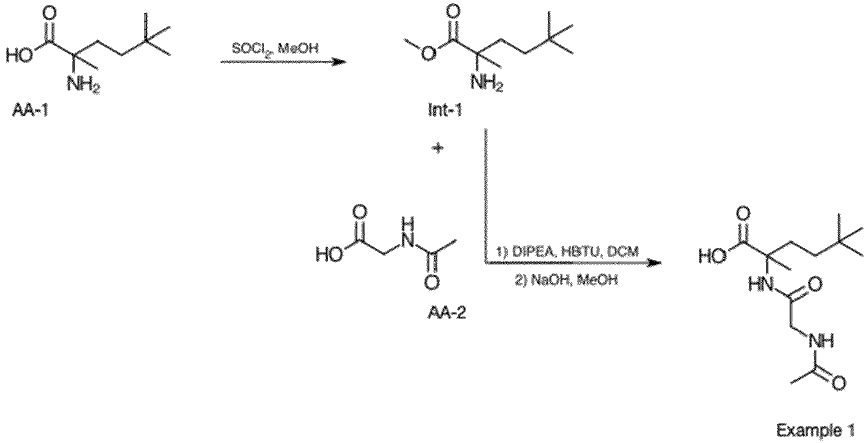

### Synthesis of methyl 2-amino-2,5,5-trimethylhexanoate hydrochloride (Int-2)

To an ice-cooled solution of 2-amino-2,5,5-trimethylhexanoic acid hydrochloride **(AA-1,** 250 mg, 1.192 mmol) in methanol (1 ml) under argon atmosphere was added dropwise thionyl chloride (0.261 ml, 3.58 mmol, 3.0 equiv.). The mixture was stirred at 0°C for 15 min and then at room temperature for 3 hours. A white thick suspension had formed. The suspension was diluted with little MeOH and stirred overnight. Thionyl chloride (0.261 ml, 3.58 mmol) was added additionally and after addition the mixture was heated under reflux for 4.5 hours. The mixture was allowed to cool to rt and solvent evaporated, MeCN added and solvent evaporated, affording methyl 2-amino-2,5,5-trimethylhexanoate hydrochloride **(Int-1,** 233 mg, 1.041 mmol, 87% yield) as an off-white turbid oil. LCMS (Method 1, 1.782 min; M+H-HCI = 188.1; calcd. 188.2). ¹H-NMR (400 MHz, DMSO) δ 8.53 (s, 2H), 3.77 (s, 3H), 1.83 - 1.70 (m, 2H), 1.47 (s, 3H), 1.33 - 1.20 (m, 1H), 1.06 - 0.93 (m, 1H), 0.85 (s, 9H).

### Synthesis of 2-[(acetylaminomethyl)carbonylamino]-2,5,5-trimethyl hexanoic acid. Example 1

HBTU (202 mg, 0.534 mmol), acetyl glycine **(AA-2,** 62.5 mg, 0.534 mmol) and DIPEA (0.093 ml, 0.534 mmol) were added to a solution of methyl 2-amino-2,5,5-trimethylhexanoate (100 mg, 0.534 mmol) in dichloromethane (10 ml). The mixture was stirred at room temperature overnight. The resulting suspension was washed with 1M aqueous hydrochloric acid (10 mL), saturated aqueous sodium bicarbonate (3 × 10 mL) and water (10 mL). The organic layer was dried over sodium sulphate and concentrated in vacuo. The residue was taken up in methanol (1 mL) and 1M aqueous sodium hydroxide (500 µL) was added. The mixture was stirred overnight at room temperature. The mixture was submitted for basic HPLC preparative purification (method 2). Fractions were combined and dried in vacuo to afford 2-(2-acetamidoacetamido)-2,5,5-trimethylhexanoic acid (21.5 mg, 0.079 mmol, 14 % yield, >90% purity). LCMS (Method 1, 0.618 min; M+H = 273.4; calcd. 273.2). ¹H-NMR (400 MHz, DMSO) δ 8.18 (t, J = 5.9 Hz, 1H), 7.85 (s, 1H), 3.60 (d, J = 5.9 Hz, 2H), 1.98 - 1.86 (m, 1H), 1.85 (s, 3H), 1.69 - 1.56 (m, 1H), 1.32 (s, 3H), 0.99 (tq, J = 13.5, 6.3 Hz, 2H), 0.80 (s, 9H).

The following Examples 2-5 in Table 1 below were prepared in an analogous manner to Example 1, starting from the corresponding acid.

**Table 1**

| Example | Structure | Yield | LCMS | NMR |
|---|---|---|---|---|
| **2** | 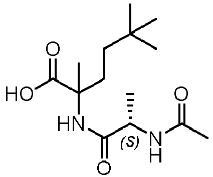 | 18.7 mg (0.06 5 mmol , 12% | Method 1, 0.668 min; M+H = 287.4; | ¹H-NMR (400 MHz, DMSO) δ 8.16 - 8.03 (m, 1H), 7.94 - 7.83 (m, 1H), 4.26 - 4.09 (m, 1H), 1.98 - 1.85 (m, 1H), 1.83 (d, J = 1.9 Hz, 3H), 1.69 - 1.55 (m, 1H), |
| | | yield, >90% purity ) | calcd. 287.2 | 1.31 (s, 3H), 1.16 (d, J = 7.0 Hz, 3H), 1.02 - 0.89 (m, 2H), 0.79 (s, 9H). |
| **3** | 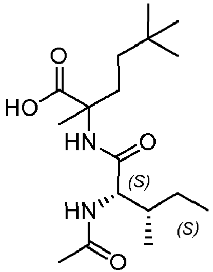 | 14.0 mg (0.04 3 mmol , 7% yield, >90% purity ) | Method 1, 0.867 min; M+H = 329.4; calcd. 329.2 | ¹H-NMR (400 MHz, DMSO) δ 7.98 - 7.78 (m, 2H), 4.29 - 4.05 (m, 1H), 1.93 - 1.60 (m, 6H), 1.49 - 1.37 (m, 1H), 1.32 (s, 3H), 1.15-0.91 (m, 3H), 0.81 (d, J = 8.0 Hz, 15H). |
| **4** | 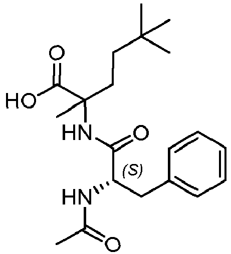 | 70.4 mg (0.19 4 mmol , 18% yield, 97.75 % purity ) | Method 1, 0.914 min; M-H = 361.4; calcd. 361.2 | ¹H-NMR (400 MHz, DMSO) δ 8.29 - 8.16 (m, 1H), 7.99 (d, J = 4.7 Hz, 1H), 7.27 - 7.21 (m, 4H), 7.21 - 7.13 (m, 1H), 4.46 - 4.28 (m, 1H), 3.05 - 2.97 (m, 1H), 2.68 (dd, J = 13.8, 10.0 Hz, 1H), 2.05 - 1.87 (m, 1H), 1.75 (s, 3H), 1.68 - 1.55 (m, 1H), 1.31 (s, 3H), 1.08 - 0.88 (m, 2H), 0.79 (d, J = 6.9 Hz, 9H). |
| **5** | 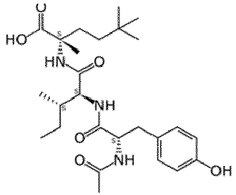 | | | |

### Method information

Method 1: Apparatus: Waters IClass; Bin. Pump: UPIBSM, SM: UPISMFTN with SO; UPCMA, PDA: UPPOATC, 210-320 nm, SQD: ACQ-SQD2 ESI; ELSD: gaspressure 40 psi, drift tube temp: 50°C; column: Waters XSelect CSH C18, 50×2.1mm, 2.5µm, Temp: 25°C, Flow: 0.6 mL/min, Gradient: t0 = 5% B, t2.0min = 98% B, t2.7min = 98% B, Posttime: 0.3 min, Eluent A: 10mM ammonium bicarbonate in water (pH=9.5), Eluent B: acetonitrile.

Method 2: Apparatus: Agilent 1260 Infinity, 1260 G1312B Bin. Pump, 1260 G1367E WPS, 1260 TCC G1316A Column Comp. 1260 G1315C DAD (210-320 nm, 210 and 220nm), PDA (210-320 nm), G6130B MSD ESI pos/neg (mass range 100-1000), Column: Waters XSelect CSH C18 (30×2.1mm 3.5µ), Flow: 1 ml/min; Column Temp: 25°C, Eluent A: 10 mM ammonium bicarbonate in water (pH 9), Eluent B: Acetonitrile, Gradient: t=0 min 5% B, t=1.6 min 98% B, t=3 min 98% B, Postrun: 1.3 min.

### BIOLOGICAL DATA

### Neurotensin scintillation proximity assay

The compounds of the invention could be tested in a Neurotensin (NTS) scintillation proximity assay (SPA), NTS, which is a 13 amino acid neuropeptide, is a sortilin ligand. The IC₅₀ data obtained is a measure of the amount of the compound required to inhibit the binding of NTS to sortilin by 50%. The skilled person will recognise that the lower the IC₅₀ value, the less of the compound needed to achieve the desired effect, and as a result, the chances of undesirable off-target effects are reduced.

Compound affinity can be determined by measuring the displacement of [³H]-neurotensin binding to *h*-Sortilin in SPA format. Total volume of 40 µl in 50 mM HEPES pH 7.4 assay buffer containing 100 mM NaCl, 2.0 mM CaCl2, 0.1% BSA and 0.1% Tween-20. Compound pre-incubation for 30 minutes at room temperature with 150 nM of 6his-Sortilin before 5 nM [3H]-Neurotensin and Ni chelate imaging beads (Perkin Elmer) may be added, after 6 hours the plate may be read on a ViewLux with 360 s exposure time. Dose-response evaluation of compounds can be performed with 8 concentrations of drugs (covering 3 decades). IC₅₀ values can be calculated by nonlinear regression using the sigmoid concentration-response (variable slope) using CDD Vault software.

### Human Sortilin GCI binding assay

The exemplified compounds of the invention were tested in a Grating-Coupled Interferometry (GCI) Sortilin binding assay. The GCI data is shown in Table 2 below. The Equilibrium dissociation constant (K_{D}) is a measure of the propensity of a complex (i.e: a ligand and receptor) to dissociate and is used to describe the affinity of an interaction between the constituents of the complex (i.e: a ligand and receptor). The skilled person will recognise that the lower the K_{D} value, the less of the compound needed to achieve the desired effect, and as a result, the chances of undesirable off-target effects are reduced.

Compound affinity was determined by measuring the association and dissociation of compounds to Human (6-His)Sortilin (RnD Systems) using a Creoptix Wave instrument (Creoptix - Malvern Panalytical). Both Human (6-His)Sortilin immobilisation and compound evaluation were performed in HBS-N running buffer: 10 mM HEPES pH 7.4, 150 mM NaCl, 1 % DMSO - Cytiva which was filtered and degassed for 15 minutes before use.

A 4PCH WAVEchip (Creoptix - Malvern Panalytical) was conditioned prior to immobilisiation using 0.2x HBS-N running buffer with 0.1 M Borate pH 9.0, 1 M NaCl (Xantec Bioanalytics) injected across all flow cells. A buffer exchange was performed to 1x HBS-N running buffer and Human (6-His)Sortilin was immobililised in pH 5.0 Acetate solution (Cytiva) via amine coupling following EDC/NHS (Cytiva) surface activation and then passivated via injection of 50 mM Tris pH 7.4.

Compounds were tested in 'WAVE-RAPID' assays at a single concentration (usually: 1 µM, 10 µM or 100 µM) in time-response format (short pulses of increasing duration) using either 'Weak-Binders' (Settings: 400 µl/min flow rate, 40 Hz acquisiton rate, 5 seconds association time and 20 seconds dissociation time) or 'Intermediate Binders' (Settings: 100 µl/min flow rate, 10 Hz acquisiton rate, 25 seconds association time and 300 seconds dissociation time) settings. Which was deemed most appropriate was based upon the affinity and kinetics of the compound tested.

DMSO calibration was performed as per manufacturers requirements by injection of running buffer containing DMSO 0.5% above that of the running buffer DMSO (1.5 % in that instance) every 20 cycles. All data was double referenced (blank and reference-channel subtracted) with K_{D} values calculated by fitting to a `1:1 kinetic' binding model using 'WAVEcontrol' software (Creoptix - Malvern Panalytical).

**Table 2**

| **Example** | **h-Sortilin GCI K_{D} (µM)** |
|---|---|
| 1 | 12.4 |
| 2 | 4.64 |
| 3 | 1.30 |
| 4 | 2.00 |

### REFERENCES

1. Tauris, J., et al., Proneurotrophin-3 May Induce Sortilin-Dependent Death In Inner Ear Neurons. Eur J Neuroscience (2020), 33(4), pp.622-31.
2. Goettsch, C., et al., Sortilin and Its Multiple Roles in Cardiovascular and Metabolic Diseases. Atherosclerosis, Thrombosis and Vascular Biology (2017), 38(1), pp. 19-25.
3. Willnow, T.E., et al., Sortilins: new players in lipoprotein metabolism. Current Opinion in Lipidology (2011), 22(2), pp. 79-85.
4. Kjolby, M., et al., Sort1, encoded by the cardiovascular risk locus 1p13.3, is a regulator of hepatic lipoprotein export. Cell Metabolism (2010), 12(3), pp. 213-223.
5. Jansen, P., et al., Roles for the pro-neurotrophin receptor sortilin in neuronal development, aging and brain injury. Nature Neuroscience (2007), 10(11), pp. 1449-1457.
6. Tenk, H.K., et al., ProBDNF induces neuronal apoptosis via activation of a receptor complex of p75NTR and sortilin. J Neuroscience (2005), 10(11), pp. 1449-1457.
7. Nykjaer, A., et al., Sortilin is essential for proNGF-induced neuronal cell death. Nature (2004), 427(6977), pp.843-848.
8. Huang, G. et al., Insulin responsiveness of glucose transporter 4 in 3T3-L1 cells depends on the presence of sortilin. Mol Biol Cell (2013), 24(19), pp.3115-3122.
9. Pan, X. et al., Sortilin and retromer mediate retrograde transport of Glut4 in 3T3-L1 adipocytes. Mol Biol Cell (2017), 28(12), pp.1667-1675.
10. Kaddai, V. et al. Involvement of TNF-α in abnormal adipocyte and muscle sortilin expression in obese mice and humans. Diabetologia (2009) 52, pp. 932-940.
11. Mortensen, M.B. et al., Targeting sortilin in immune cells reduces proinflammatory cytokines and atherosclerosis. J Clin Invest (2014), 124(12), pp. 5317-5322.
12. Shi, J. & Kandror, K. V., Sortilin Is Essential and Sufficient for the Formation of Glut4 Storage Vesicles in 3T3-L1 Adipocytes. Developmental Cell (2005), 9, pp. 99-108.
13. Gao, A. et al., Implications of Sortilin in Lipid Metabolism and Lipid Disorder Diseases. DNA and Cell Biology (2017), 36(12), pp. 1050-1061.
14. Oh, T.J. et al., Circulating sortilin level as a potential biomarker for coronary atherosclerosis and diabetes mellitus. Cardiovascular Diabetology (2017), 16(92).
15. Skeldal, S. et al., Mapping of the Interaction Site between Sortilin and the p75 Neurotrophin Receptor Reveals a Regulatory Role for the Sortilin Intracellular Domain in p75 Neurotrophin Receptor Shedding and Apoptosis. J Biol Chem (2012), 21(287), pp. 43798-43809.
16. Wuts, P.G.M. and Greene, T.W, Greene's Protective Groups in Organic Synthesis, 4th Edition, John Wiley and Sons, New York (2006).
17. Andersen, K R et al., Introducing site-specific cysteines into nanobodies for mercury labelling allows de novo phasing of their crystal structures. Acta Crystallographia Section. D (2017), 73(1), pp. 804-813.
18. Kabsch, W, XDS. Acta Crystallographia Section. D (2010),66(2), pp. 125-132.
19. Afonine, P V et al., Acta Crystallographia Section. D (2012), 68(4), pp. 352-367.
20. Emsley, P. et al., Acta Crystallographia Section. D (2010),66(4), pp. 486-501.

### Sequences referenced throughout the specification and forming part of the description

SEQ ID NO: 1 (full length sortilin- isoform 1) 
1   
51   
101 
151 
201 
251 
301 
351 
401 
451 
501 
551 
601 
651 
701 
751 
801 
SEQ ID NO: 2 (full length sortilin- isoform 2) 
1 MERPWGAADG LSRWPHGLGL LLLLQLLPPS TLSQDRLDAP PPPAAPLPRW
51 SGPIGVSWGL RAAAAGGAFP RGGRWRRSAP GEDEECGRVR DFVAKLANNT
101 HQHVFDDLRG SVSLSWVGDS TGVILVLTTF HVPLVIMTFG QSKLYRSEDY
151 GKNFKDITDL INNTFIRTEF GMAIGPENSG KVVLTAEVSG GSRGGRIFRS
201 SDFAKNFVQT DLPFHPLTQM MYSPQNSDYL LALSTENGLW VSKNFGGKWE
251 EIHKAVCLAK WGSDNTIFFT TYANGSCTDL GALELWRTSD LGKSFKTIGV
301 KIYSFGLGGR FLFASVMADK DTTRRIHVST DQGDTWSMAQ LPSVGQEQFY
351 SILAANDDMV FMHVDEPGDT GFGTIFTSDD RGIVYSKSLD RHLYTTTGGE
401 TDFTNVTSLR GVYITSVLSE DNSIQTMITF DQGGRWTHLR KPENSECDAT
451 AKNKNECSLH IHASYSISQK LNVPMAPLSE PNAVGIVIAH GSVGDAISVM
501 VPDVYISDDG GYSWTKMLEG PHYYTILDSG GIIVAIEHSS RPINVIKFST
551 DEGQCWQTYT FTRDPIYFTG LASEPGARSM NISIWGFTES FLTSQWVSYT
601 IDFKDILERN CEEKDYTIWL AHSTDPEDYE DGCILGYKEQ FLRLRKSSVC
651 QNGRDYVVTK QPSICLCSLE DFLCDFGYYR PENDSKCVEQ PELKGHDLEF
701 CLYGREEHLT TNGYRKIPGD KCQGGVNPVR EVKDLKKKCT SNFLSPEKQN
751 SKSNSVPIIL AIVGLMLVTV VAGVLIVKKY VCGGRFLVHR YSVLQQHAEA
801 NGVDGVDALD TASHTNKSGY HDDSDEDLLE
SEQ ID NO: 3 (mature sortilin)

1 MTFGQSKLYR SEDYGKNFKD ITDLINNTFI RTEFGMAIGP ENSGKVVLTA
51 EVSGGSRGGR IFRSSDFAKN FVQTDLPFHP LTQMMYSPQN SDYLLALSTE
101 NGLWVSKNFG GKWEEIHKAV CLAKWGSDNT IFFTTYANGS CTDLGALELW
151 RTSDLGKSFK TIGVKIYSFG LGGRFLFASV MADKDTTRRI HVSTDQGDTW
201 SMAQLPSVGQ EQFYSILAAN DDMVFMHVDE PGDTGFGTIF TSDDRGIVYS
251 KSLDRHLYTT TGGETDFTNV TSLRGVYITS VLSEDNSIQT MITFDQGGRW
301 THLRKPENSE CDATAKNKNE CSLHIHASYS ISQKLNVPMA PLSEPNAVGI
361 VIAHGSVGDA ISVMVPDVYI SDDGGYSWTK MLEGPHYYTI LDSGGIIVAI

401 EHSSRPINVI KFSTDEGQCW QTYTFTRDPI YFTGLASEPG ARSMNISIWG
451 FTESFLTSQW VSYTIDFKDI LERNCEEKDY TIWLAHSTDP EDYEDGCILG
501 YKEQFLRLRK SSVCQNGRDY VVTKQPSICL CSLEDFLCDF GYYRPENDSK
551 CVEQPELKGH DLEFCLYGRE EHLTTNGYRK IPGDKCQGGV NPVREVKDLK
601 KKCTSNFLSP EKQNSKSNSV PIILAIVGLM LVTVVAGVLI VKKYVCGGRF
651 LVHRYSVLQQ HAEANGVDGV DALDTASHTN KSGYHDDSDE DLLE

## Claims

**1.** A compound of formula (I) 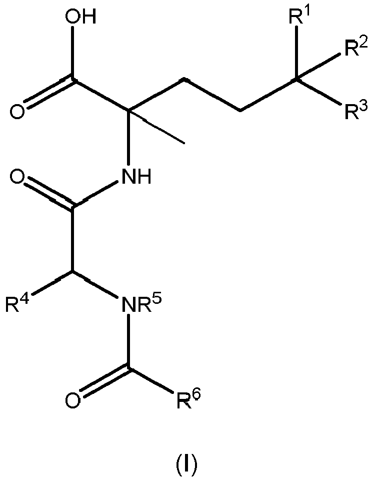
or a pharmaceutically acceptable salt, solvate, hydrate, tautomer, optical isomer, N-oxide, and/or prodrug thereof; wherein
R¹, R² and R³ are each independently selected from the group consisting of halo, H, (C₁-C₄)alkyl, halo-(C₁-C₄)alkyl, (C₂-C₄)alkenyl, and halo-(C₂-C₄)alkenyl;
R⁴ is selected from the group consisting of H, (C₁-C₁₀)alkyl, halo-(C₁-C₁₀)alkyl, (C₂-C₁₀)alkenyl, halo-(C₂-C₁₀)alkenyl, (C₃-C₂₀)aryl, halo-(C₃-C₂₀)aryl, (C₃-C₈)heteroaryl, halo-(C₃-C₂₀)heteroaryl, (C₁-C₆)-alkylene-(C₃-C₂₀)-aryl, (C₁-C₆)-alkylene-(C₃-C₂₀)-heteroaryl, (C₁-C₆)-alkylene-(3- to 10- membered-heterocyclic ring);
wherein the aryl group in (C₁-C₆)-alkylene-(C₃-C₂₀)-aryl, the heteroaryl group in (C₁-C₆)-alkylene-(C₃-C₂₀)-heteroaryl or the heterocyclic ring in (C₁-C₆)-alkylene-(3- to 10- membered heterocyclic ring) is optionally substituted with one or more substituents independently selected from halo, H, -OH, (C₁-C₄)alkyl, halo-(C₁-C₄)alkyl, (C₁-C₄)alkoxy and halo-(C₁-C₄)alkoxy;
R⁵ is selected from the group consisting of H, (C₁-C₄)alkyl, halo-(C₁-C₄)alkyl, (C₂-C₄)alkenyl and halo-(C₁-C₄)alkenyl;
wherein R⁶ is selected from:
(a) the group consisting of (C₁-C₆)alkyl, halo-(C₁-C₆)alkyl, (C₂-C₆)alkenyl, halo-(C₂-C₆)alkenyl, (C₃-C₂₀)aryl, halo-(C₃-C₂₀)aryl, (C₃-C₂₀)heteroaryl and halo-(C₃-C₂₀)heteroaryl; or
(b) 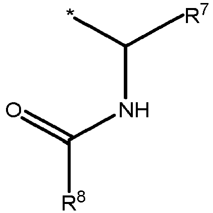
wherein R⁷ is selected from the group consisting of H, (C₁-C₁₀)alkyl, and (C₂-C₁₀)alkenyl;
wherein the alkyl and alkenyl groups are optionally substituted with halo, an amide group and phenol; and
R⁸ is selected from the group consisting of (C₁-C₁₀)alkyl, halo-(C₁-C₁₀)alkyl, (C₂-C₁₀)alkenyl, halo-(C₂-C₁₀)alkenyl and 3- to 10- membered-heterocyclic ring;
wherein the heterocyclic ring is optionally substituted with one or more substituents independently selected from halo, -OH, (C₁-C₄)alkyl, halo-(C₁-C₁₀)alkyl, acetyl, (C₁-C₄)alkoxy, and halo-(C₁-C₄)alkoxy.

**2.** The compound according to claim 1, wherein R¹, R² and R³ are each independently selected from the group consisting of halo, (C₁-C₂)alkyl and halo-(C₁-C₂)alkyl.

**3.** The compound according to claim 1 or claim 2, wherein R¹, R² and R³ are each independently selected from F, CH₃ and CF₃.

**4.** The compound according to any preceding claim, wherein R⁴ is selected from the group consisting of H, (C₁-C₆)alkyl, halo-(C₁-C₆)alkyl, (C₃-C₁₀)aryl, (C₁-C₃)-alkylene-(C₃-C₁₀)-aryl, (C₁-C₃)-alkylene-(C₃-C₂₀)-heteroaryl and (C₁-C₃)-alkylene-(3- to 10- membered-heterocyclic ring);
wherein the aryl group in (C₁-C₃)-alkylene-(C₃-C₁₀)-aryl, the heteroaryl group in (C₁-C₃)-alkylene-(C₃-C₁₀)-heteroaryl or the heterocyclic ring in (C₁-C₃)-alkylene-(3- to 10- membered heterocyclic ring) is optionally substituted with one or more substituents independently selected from halo, H, -OH, (C₁-C₄)alkyl, halo-(C₁-C₄)alkyl, (C₁-C₄)alkoxy and halo-(C₁-C₄)alkoxy.

**5.** The compound according to any preceding claim, wherein R⁶ is (a) and R⁴ is selected from the group consisting of:
(i) H
(ii) CH₃ 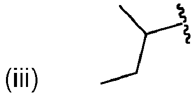 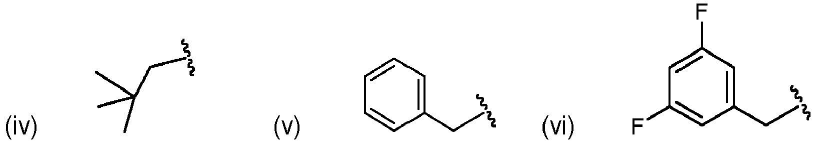 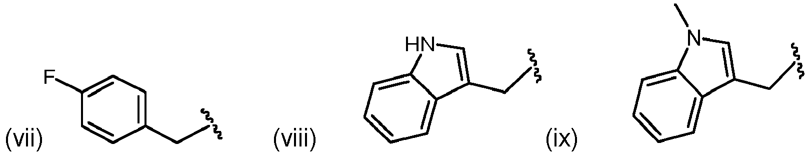 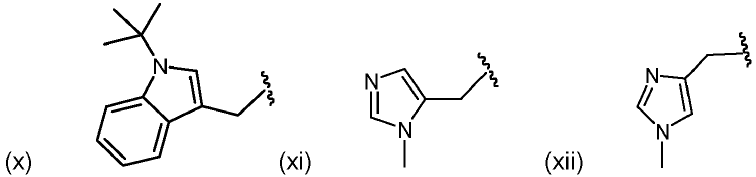 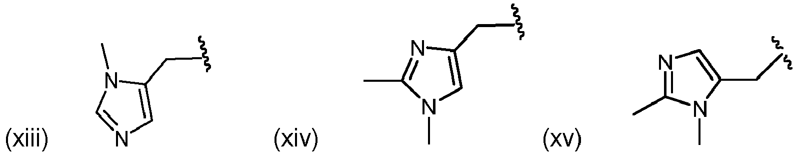 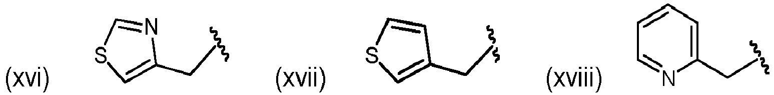 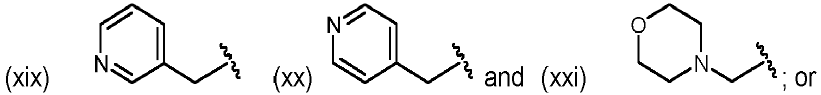
wherein R⁶ is (b) and R⁴ is selected from:

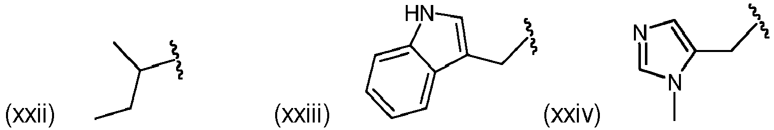

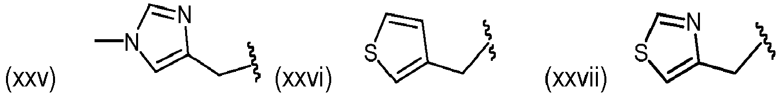

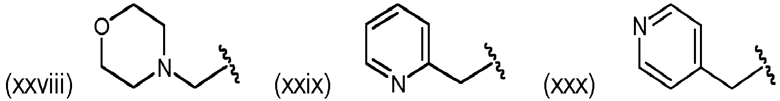

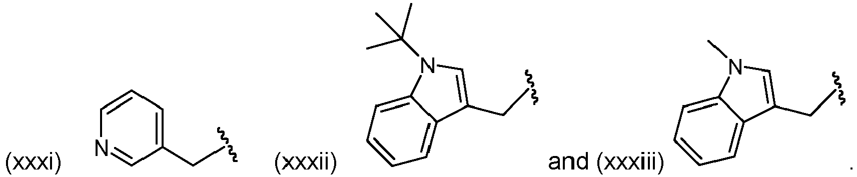

**6.** The compound according to any preceding claim, wherein R⁵ is selected from the group consisting of H, (C₁-C₃)-alkyl and (C₁-C₃)haloalkyl.

**7.** The compound according to any preceding claim, wherein R⁵ is selected from the group consisting of H and CH₃, preferably wherein R⁵ is H.

**8.** The compound according to any preceding claim, wherein R⁶ is (b), and R⁷ is selected from the group consisting of H and (C₁-C₄)alkyl;
wherein the alkyl group is optionally substituted with halo, an amide group and phenol.

**9.** The compound according to claim 8, wherein R⁷ is selected from the group consisting of:
(i) H
(ii) CH₃ 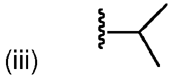 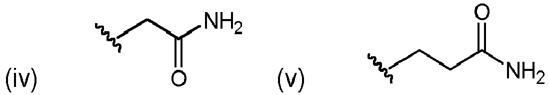
and

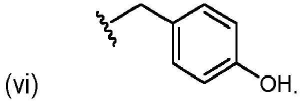

**10.** The compound according to any preceding claim, wherein R⁶ is (b) and R⁸ is selected from the group consisting of (C₁-C₃)alkyl, halo-(C₁-C₃)alkyl, and 3-to 8- membered-heterocyclic ring;
wherein the heterocyclic ring is optionally substituted with one or more substituents independently selected from halo, -OH, (C₁-C₄)alkyl and acetyl.

**10.** The compound according to claim 10, wherein R⁸ is selected from the group consisting of:
(i) CH³ 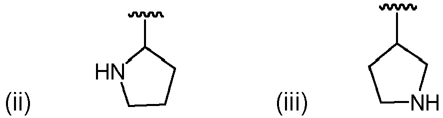 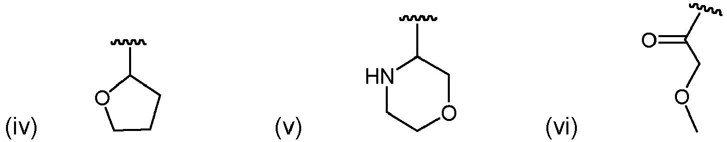 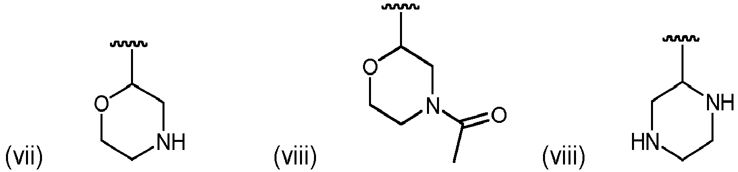
and

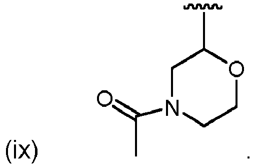

**11.** The compound according to any of claims 1 to 7, wherein R⁶ is (a) and is selected from the group consisting of (C₁-C₄)alkyl, halo-(C₁-C₄)alkyl, (C₃-C₈)heteroaryl, and halo-(C₃-C₈)heteroaryl; preferably wherein R⁶ is selected from the group consisting of CH₃, pyrazine and morpholine.

**12.** The compound according to any preceding claim, wherein the compound of Formula (I) is selected from the group consisting of:
(2S)-2-(2-acetamidoacetamido)-2,5,5-trimethylhexanoic acid;
(2S)-2-[(2S)-2-acetamidopropanamido]-2,5,5-trimethylhexanoic acid;
(2S)-2-[(2S,3S)-2-acetamido-3-methylpentanamido]-2,5,5-trimethylhexanoic acid;
(2S)-2-[(2S)-2-acetamido-3-phenylpropanamido]-2,5,5-trimethylhexanoic acid;
(2S)-2-[(2S,3S)-2-[(2S)-2-acetamido-3-(4-hydroxyphenyl)propanamido]-3-methylpentanamido]-2,5,5-trimethylhexanoic acid;
(S)-2-[(S)-2-acetylaminopropionylamino]-2,5,5-trimethylhexanoic acid;
(S)-2-[(S)-2-acetylamino-3-phenylpropionylamino]-2,5,5-trimethylhexanoic acid;
(S)-2-[(2S,3S)-2-acetylamino-3-methylvalerylamino]-2,5,5-trimethylhexanoic acid;
(S)-2-{(2S,3S)-2-[(S)-2-acetylamino-3-(p-hydroxyphenyl)propionylamino]-3-methylvalerylamino}-2,5,5-trimethylhexanoic acid; and
(S)-2-[(acetylaminomethyl)carbonylamino]-2,5,5-trimethylhexanoic acid.

**13.** A pharmaceutical composition comprising a compound according to any preceding claim and a pharmaceutically acceptable carrier, excipient, and/or diluent.

**14.** The compound according to any one of claims 1 to 12, or the pharmaceutical composition of claim 13, for use in therapy.

**15.** The compound according to any one of claims 1 to 12, or the pharmaceutical composition of claim 13, for use in the treatment or prevention of a neurodegenerative disorder, an inflammatory disorder, a lysosomal storage disorder, a cancer, pain, diabetes mellitus, diabetic retinopathy, glaucoma, uveitis, cardiovascular disease, hereditary eye conditions or hearing loss;
preferably wherein the neurodegenerative disorder is selected from frontotemporal dementia, Alzheimer's disease, Parkinson's disease and spinal cord injury;
preferably wherein the inflammatory disorder is selected from inflammatory diseases and neuroinflammation;
preferably wherein the lysosomal storage disorder is selected from the group consisting of NCL/Batten Disease caused by mutations in CLN gene *CLN1 (PPT1), CLN2 (TPP1), CLN3, CLN4 (DNAJC5), CLN5, CLN6, CLN7 (MFSD8), CLN8, CLN10 (CTSD), CLN11, CLN12 (ATP13A2), CLN13 (CTSF), CLN14 (KCTD7), CLCN6,* and/or SGSH; Pompe disease, Fabry disease, Gaucher disease, Niemann-Pick disease Types A, B, and C; GM1 gangliosidosis, GM2 gangliosidosis (including Sandhoff and Tay-Sachs), mucopolysachariddoses (MPS) types I (Hurler disease)/II (Hunter disease)/Illa (Sanfilippo A)/IIIB (Sanfilippo B)/lllc (Sanfilippo C)/Illd (Sanfilippo D)/IVA (Morqui" A)/'VB/VI/VII (Sly)/IX, mucolipisosis III (I-cell) and IV, multiple sulfatase deficiency; sialidosis, galactosialidosis, α-mannosidosis, β-mannosidosis, apartylglucosaminuria, fucosidosis, Schindler disease, metachromatic leukodystrophy caused by deficiencies in either arylsulfatase A or Saposin B, globoid cell leukodystrophy (Krabbe disease), Farber lipogranulomatosis, Wolman and cholesteryl ester storage disease, pycnodystostosis, cystinosis, Salla disease, Danon disease, Griscelli disease Types ½/3, Hermansky Pudliak Disease, and Chédiak-Higashi syndrome;
preferably wherein the cancer is selected from breast cancer, lung cancer, ovarian cancer, prostate cancer, thyroid cancer, pancreatic cancer, glioblastoma and colorectal cancer; and
preferably wherein the hearing loss is selected from noise-induced hearing loss, ototoxicity induced hearing loss, age-induced hearing loss, idiopathic hearing loss, tinnitus and sudden hearing loss.
